# EUROPEAN PATENT APPLICATION

(11) **EP 4 400 576 A1**
(43) Date of publication of application: **17.07.2024**
(21) Application number: 22867359.6
(22) Date of filing: 07.09.2022
(51) Int. Cl.: C12N 1/04, A61K 9/10, A61K 9/19, A61K 35/14, A61K 35/26, A61K 35/28, A61K 35/51, A61K 35/545, A61K 47/02, A61K 47/12, A61K 47/18, A61K 47/26, A61K 47/42, A61P 31/04, A61P 31/12, A61P 35/00, A61P 35/02, A61P 37/04

(54) **METHOD FOR TREATING CELLS**

(30) Priority: 08.09.2021 JP 2021146461
(71) Applicant: GAIA BioMedicine Inc., Fukuoka 810-0041 (JP)
(72) Inventor: HARADA Yui, Fukuoka-shi, Fukuoka 819-0395 (JP); YONEMITSU Yoshikazu, Fukuoka-shi, Fukuoka 819-0395 (JP)
(74) Representative: Vossius & Partner Patentanwälte Rechtsanwälte mbB
(86) International application number: PCT/JP2022/033487
(87) International publication number: WO 2023/038037

(57) **Abstract**

A method for thawing frozen highly active NK cells with maintaining high activity is provided. A solution for suspending highly active NK cells satisfying the following criteria is used to thaw frozen cells. A solution for suspending cells for administration to humans that satisfies the following criteria is provided: (1) containing potassium ions, (2) having a pH of 6.4 or higher, (3) not containing chloride ions at a concentration of 135 mEq/L or higher, (4) not containing calcium ions at a concentration of 0.423 mM or higher, and (5) having an osmolarity of 200 to 396 mOsm.

## Description

### Technical Field

The present invention relates to a method for treating cells for administration to humans, such as highly active NK cells.

### Background Art

NK cells are important in injuring tumor cells and virus-infected cells. In August 2017, chimeric antigen receptor (CAR) T-cell therapy was approved in the United States for the treatment of relapsed and refractory B-cell acute lymphoblastic leukemia (B-ALL) in children and young adults, and in recent years, clinical application of CAR-NK has been expanding in place of CAR-T (see Non-patent document 1).

When attempting to administer cells to a patient, the first consideration is to use cells harvested from the patient himself/herself to avoid rejection. However, depending on the patient's condition, it may be difficult to collect the necessary amount of cells for treatment. In addition, the degree to which cells can be activated and proliferated in vitro varies among individuals, and there are also cases in which proliferation and activation are difficult. In addition, it takes a certain period of time for cells to be activated and proliferated, posing a problem that it is difficult to start treatment immediately. In this respect, it would be desirable if cells could be activated in advance and stored for preparation of administration.

As a method for preserving cells, it is known to preserve cells in suspension without freezing for short-time preservation (e.g., Patent document 1), and to freeze cells for long time preservation (e.g., Patent document 2). Furthermore, use of a solution for freezing containing a sodium salt, potassium salt, sugar, cryoprotectant, and hydrogencarbonate and/or carbonate has been investigated, since use of such a solution may provide cells that maintain high viability even after freeze-thawing (Patent document 3).

Also known is a method for preserving cells that are difficult to preserve, such as NK cells, in which the cells are stored in a solution containing a sodium salt, potassium salt, sugar, and protein as active ingredients (Patent document 4). Further, a cell preservation solution for refrigerated preservation containing potassium ions and lactate ions, and having an osmolarity of 200 to 350 mOsm/kg and pH of 6.0 to 8.0 is known (Patent document 5), and this preservation solution contains 0.5 to 150 mM of sugars and 1 to 4 mM of calcium ions. Furthermore, there is also known a cell or tissue preservation solution having an osmolarity of 270 to 450 mOsm/l and pH of 7 to 8, and containing K⁺ and organic acid anions (Patent document 6). In this reference, lactic acid is exemplified as the organic acid. Furthermore, it is known to use a physiological aqueous solution as a solution for suspending mammalian cells such as NK cells, and it is known that isotonic (250 to 380 mOsm/1) aqueous solutions such as Ringer's solution (lactated Ringer's solution) can be used as the physiological aqueous solution (Patent document 7). In an example of this reference, a lactated Ringer's solution ("Lactec Injection" produced by Otsuka Pharmaceutical Factory) is used (paragraph 0038), which contains potassium and lactic acid, has a pH of 6.0 to 7.5, and does not contain glucose.

### Prior Art References

### Patent documents

Patent document 1: Japanese Patent Publication (Kokai) No. 2006-230396
Patent document 2: Japanese Patent Publication (Kokai) No. 2002-233356
Patent document 3: WO2011/021618
Patent document 4: WO2013/115322
Patent document 5: Japanese Patent No. 4947948
Patent document 6: WO2002/001952
Patent document 7: Japanese Patent Publication (Kokai) No. 2013-233102
Patent document 8: WO2021/177279 (PCT/JP2021/007863, unpublished on the priority date of the present application)

### Non-patent document

Non-patent document 1: Liu E., et al., N. Engl. J. Med., 2020;382:545-53

### Summary of the Invention

### Object to be achieved by the invention

However, conventional freezing methods using protective agents such as dimethyl sulfoxide can preserve T-cell lines and primary NK cells, but are not sufficient for NK cells with high cytotoxic activity, whose activity and viability are significantly reduced by freeze-thaw operations. This problem could not be solved by use of a programmed freezer, cell density control, addition of dextran, albumin, carboxylated poly-L-lysine, etc. In addition, if cells for administration are packaged on the assumption that a certain amount of the cells will die, a step for reactivation culture is required after thawing, and the resulting cells must be further washed. Therefore, frozen and stocked highly active NK cells currently require use of facilities for cell culture and processing that meet certain criteria for handling cells for clinical use (Cell Processing Center, CPC).

By the way, the inventors of the present invention have studied methods for freezing and thawing highly active NK cells (Patent document 8). In these studies, it was found that even if viability of thawed cells is maintained high, high cytotoxic activity may not be maintained. It was also suggested that if thawed cells maintain high cytotoxic activity, viability may also be high, and it was found that high cytotoxic activity evaluated by a given method is a useful evaluation criterion for thawed cells.

An object of the present invention is to provide a thawing method effective for highly active cells. More specifically, it is to provide a method for thawing frozen highly active NK cells so that the cells maintain a cytotoxic activity of 60% or higher, preferably 80% or higher, as evaluated by a given method.

### Means for achieving the object

The inventors of the present invention found that composition of a solution used for dilution at the time of thawing frozen cells is particularly important. They also found that the composition of the solution used for such dilution can consist of only the ingredients used in infusion solutions etc. approved under the Act on Securing Quality, Efficacy and Safety of Products Including Pharmaceuticals and Medical Devices (Act No. 145 of 1960) in Japan, and accomplished the present invention.

The present invention provides the followings.
[1] A solution for suspending cells for administration to humans, which satisfies the following criteria:
   (1) containing potassium ions,
   (2) having a pH of 6.4 or higher,
   (3) not containing chloride ions at a concentration of 135 mEq/L or higher,
   (4) not containing calcium ions at a concentration of 0.423 mM or higher, and
   (5) having an osmolarity of 200 to 396 mOsm.
[2] The solution according to 1, which is for diluting a frozen product containing cells for administration to humans or a thawed product thereof.
[3] The solution according to 1 or 2, which
   contains potassium ions at a concentration of 4.00 mEq/L or higher,
   does not contain calcium ions, and
   has a pH of 7.0 to 8.3.
[4] A pharmaceutical composition containing a population of cells for administration to humans suspended in the solution according to any one of 1 to 3, wherein the cells for administration to humans are highly active NK cells.
[5] The pharmaceutical composition according to 4, wherein the population of highly active NK cells has undergone a freezing step.
[6] The pharmaceutical composition according to 5, wherein the population of highly active NK cells before freezing was collected by using a medium containing albumin, transferrin, insulin, and IL-2.
[7] A method for providing a pharmaceutical composition for infusion containing cells for administration to humans, the method comprising:
   (1) washing the cells with a medium for animal cell culture;
   (2) optionally washing and suspending the cells in a cryopreservation solution;
   (3) freezing and preserving (stocking) the cells suspended in the cryopreservation solution;
   (4) thawing the cryopreserved cells; and
   (5) suspending the thawed cells in the solution according to any one of 1 to 3 to prepare a pharmaceutical composition for infusion.

The present invention also provides the followings.
[1] A solution for suspending cells for administration to humans, which satisfies the following criteria:
   (1) containing potassium ions,
   (2) having a pH of 6.4 or higher,
   (3) not containing chloride ions at a concentration of 135 mEq/L or higher,
   (4) not containing glucose at a concentration of 5.55 mM or higher,
   (5) not containing calcium ions at a concentration of 0.423 mM or higher, and
   (6) having an osmolarity of 200 to 396 mOsm.
[2] The solution according to 1, which is for diluting a frozen product containing cells for administration to humans or a thawed product thereof.
[3] The solution according to 1 or 2, which
   contains potassium ions at a concentration of 4.00 mEq/L or higher,
   does not contain calcium ions,
   does not contain glucose, and
   has a pH of 7.0 to 8.3.
[4] A pharmaceutical composition containing a population of cells for administration to humans suspended in the solution according to any one of 1 to 3, wherein the cells for administration to humans are highly active NK cells.
[5] The pharmaceutical composition according to 4, wherein the population of highly active NK cells has undergone a freezing step.
[6] The pharmaceutical composition according to 5, wherein the population of highly active NK cells before freezing was collected by using a medium containing albumin, transferrin, insulin, and IL-2.
[7] A method for providing a pharmaceutical composition for infusion containing cells for administration to humans, the method comprising:
   (1) washing the cells with a medium for culturing animal cells;
   (2) optionally washing and suspending the cells in a cryopreservation solution;
   (3) freezing and preserving (stocking) the suspended cells in the cryopreservation solution;
   (4) thawing the cryopreserved cells; and
   (5) suspending the thawed cells in the solution according to any one of 1 to 3 to prepare a pharmaceutical composition for infusion.

### Brief Description of the Drawings

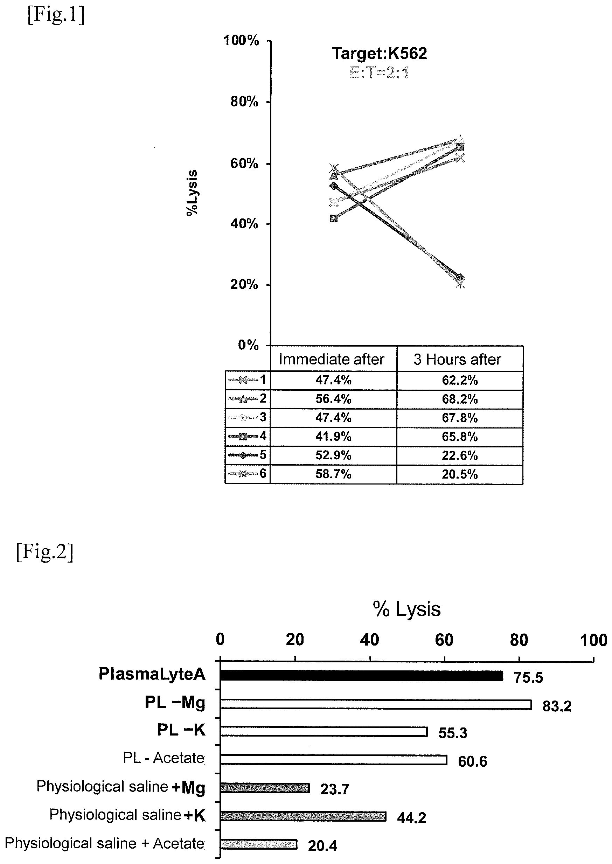

[Fig. 1] Cytotoxic activity of highly active NK cells after freezing and thawing.
   There were set a group of (1) a solution having a composition based on the concentrations of the ingredients of Plasma-Lyte A, but not containing sodium gluconate, a group of (2) a solution having a composition based on the concentrations of the ingredients of Plasma-Lyte A, but not containing sodium gluconate, and containing the other ingredients increased by 16% each to adjust osmolarity, a group of (3) a solution having a composition based on the concentrations of the ingredients of Plasma-Lyte A, but not containing sodium gluconate and having an osmolarity adjusted with NaCl, a group of (4) Plasma-Lyte A as a positive control, a group of (5) physiological saline, and a group of (6) physiological saline containing sodium gluconate at the same concentration as that of Plasma-Lyte A. Marked decreases in the activity were observed in the groups of (5) and (6) at 3 hours after the thawing and dilution.
[Fig. 2] Cytotoxic activity of highly active NK cells after freezing and thawing.
   There were set groups of (1) to (3) solutions having a composition based on the concentrations of the ingredients of Plasma-Lyte A, but not containing sodium gluconate, from which each of Mg²⁺, K⁺, and CH₃COO⁻ (acetate) was excluded, respectively, and groups of (4) to (6) physiological saline supplemented with each of the ingredients, respectively. At 3 hours after the thawing and dilution, decreases in the activity were observed in the groups (2) and (3) wherein K⁺ and CH₃COONa were excluded, respectively, but such decrease was not observed in the group (1) wherein Mg⁺ was excluded. As a result of the experiments of the groups (4) to (6), contribution to the activity by each ingredient alone was observed only for K⁺.

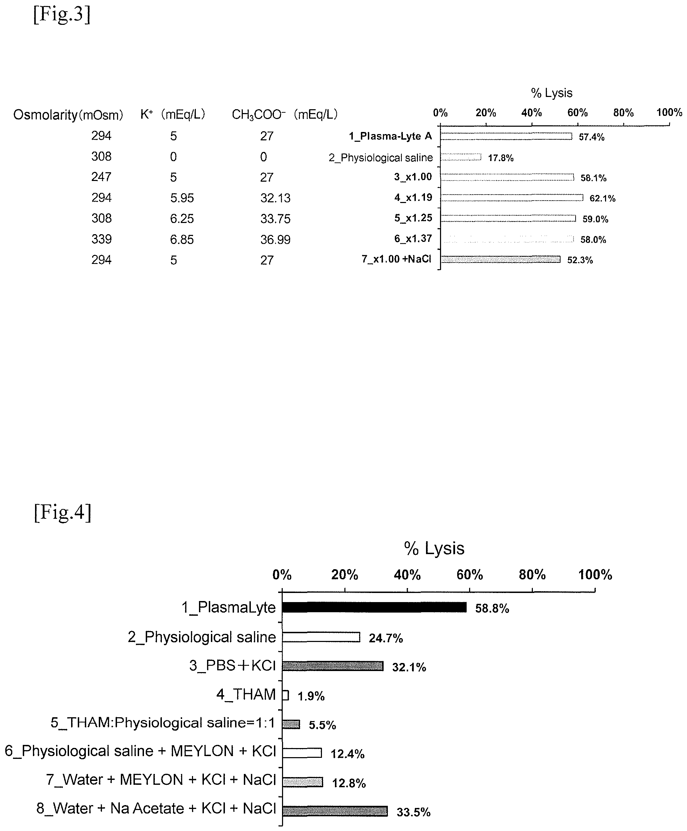

[Fig. 3] Cytotoxic activity of highly active NK cells after freezing and thawing.
   There were prepared (1) Plasma-Lyte A as a positive control, (2) physiological saline as a negative control, and (3) to (6) solutions having a composition based on the concentrations of the ingredients of Plasma-Lyte A, but not containing sodium gluconate and magnesium chloride, which were prepared to have osmolarity ratios to physiological saline of 0.80, 0.95, 1.0, and 1.10, respectively. There was also set a group of (7) a solution having a composition based on the concentrations of the ingredients of Plasma-Lyte A, of which osmolarity ratio was adjusted to 1.0 with the content of NaCl alone. No effect on the activity was observed with the solutions of (3) to (6), but an activity-decreasing tendency was observed in the group of (7), in which osmolarity ratio of the solution was adjusted to 1.0 with the content of NaCl alone.
[Fig. 4] Cytotoxic activity of highly active NK cells after freezing and thawing.
   There were set groups of (1) Plasma-Lyte A as a positive control, and (2) physiological saline as a negative control, and (3) phosphate buffer supplemented with K⁺ for a group of phosphate buffer having a K⁺ concentration of 5 mEq/L, (4) THAM Injection (THAM), (6) a solution containing physiological saline/MEYLON/KCI, and (7) a solution containing water/MEYLON/KCl/NaCl were prepared. In addition, there were also prepared (5) a solution containing THAM/MEYLON prepared so as to have a K⁺ concentration of 2.5 mEq/L, and (8) a solution containing water/CH₃COONa/KCl/NaCl. Although an increase in the activity was observed with the solution of (3) compared with that observed with (2) physiological saline, it could not sufficiently maintain the activity. The solutions of (5) and (8) also could not sufficiently maintain the activity.

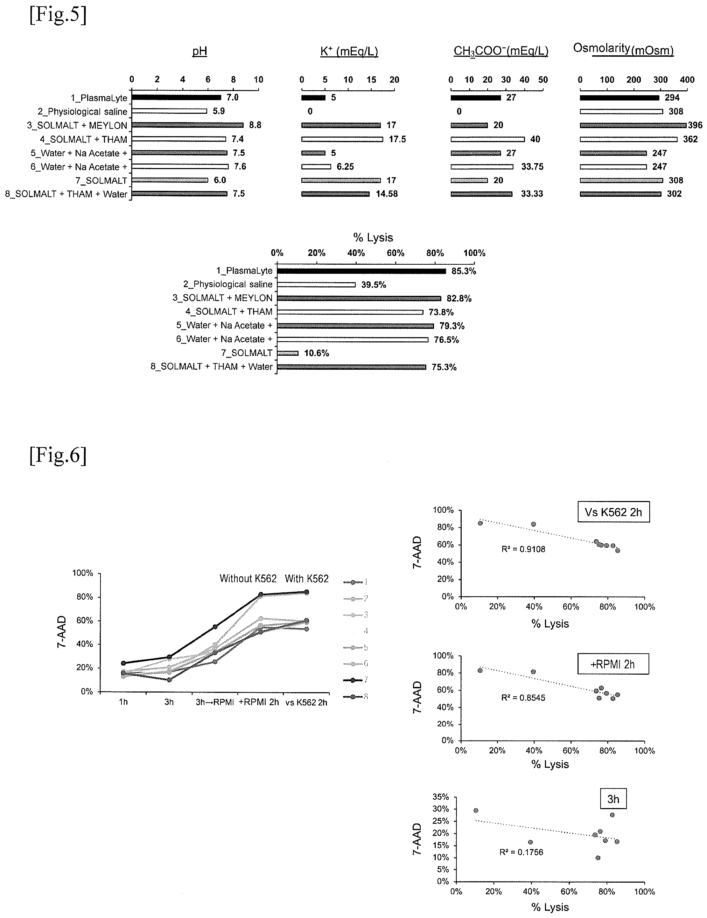

[Fig. 5] Cytotoxic activity of highly active NK cells after freezing and thawing.
   There were set groups of (1) Plasma-Lyte A as a positive control, and (2) physiological saline as a negative control, and there were prepared (7) SOLMALT Infusion (SOLMALT), as well as (3) a solution containing SOLMALT/MEYLON, and (4) a solution containing SOLMALT/THAM, for groups of solutions which pH is adjusted based on SOLMALT. In addition, there were also prepared (5) a solution having the same composition as Plasma-Lyte A except that sodium gluconate and Mg²⁺ were both excluded, where the other ingredients were contained at the same concentrations as those in Plasma-Lyte A, and (6) a solution having the same composition as Plasma-Lyte A except that sodium gluconate and Mg²⁺ were both excluded, and only NaCl was contained in a decreased amount so that the solution had the same osmolarity as the solution of (5). Further, there was additionally set a group of (8) SOLMALT having a pH adjusted with THAM, and an osmolarity reduced by adding water after the pH adjustment. Whereas the activity could not be maintained with the solution of (7), an activity-improving tendency was confirmed with the solutions of (3) and (4). This tendency was confirmed with reproducibility, and similar results could be obtained in the group of the solution (8) SOLMALT having a pH adjusted with THAM, and an osmolarity reduced by adding water after the pH adjustment. It was also experimentally confirmed from these results that it becomes difficult to maintain the activity when pH decreases to 6.0, and acceptable results can be obtained up to pH 8.8 (Fig. 5).
[Fig. 6] Cytotoxic activity of highly active NK cells after freezing and thawing.
   There were set groups of (1) Plasma-Lyte A as a positive control, and (2) physiological saline as a negative control, and there were prepared (7) SOLMALT, as well as (3) a solution containing SOLMALT/MEYLON, and (4) a solution containing SOLMALT/THAM for groups of solutions prepared based on SOLMALT with pH adjustment. In addition, there were also prepared (5) a solution having the same composition as Plasma-Lyte A except that sodium gluconate and Mg²⁺ were both excluded, where the other ingredients were contained at the same concentrations as those in Plasma-Lyte A, and (6) a solution having the same composition as Plasma-Lyte A except that sodium gluconate and Mg²⁺ were both excluded, and only NaCl was contained in a decreased amount so that the solution had the same osmolarity as the solution of (5). It was found that decrease in viability, which was not observed immediately after the thawing and dilution, was observed after replacement with RPMI.

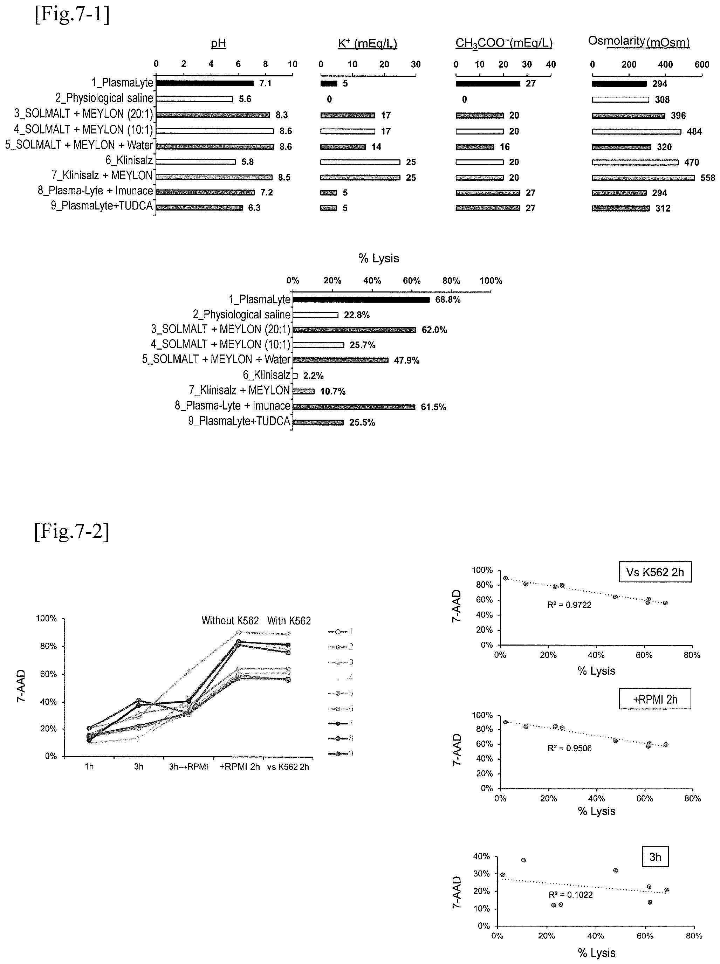

[Fig. 7-1] Cytotoxic activity of highly active NK cells after freezing and thawing.
   There were set groups of (1) Plasma-Lyte A as a positive control, and (2) physiological saline as a negative control, and there were prepared (3) a solution consisting of SOLMALT and MEYLON at a ratio of 20:1, (4) a solution consisting of SOLMALT and MEYLON at a ratio of 10: 1, and (5) a solution containing SOLMALT/MEYLON/water for groups of solutions prepared based on SOLMALT with pH adjustment with MEYLON, as well as (6) Klinisalz, and (7) a solution containing Klinisalz/MEYLON for a group of a solution prepared based on Klinisalz with pH adjustment with MEYLON. There were also prepared (8) Plasma-Lyte A + 140,000 IU/mL Imunace and (9) a solution containing Plasma-Lyte A + 875 µM tauroursodeoxycholic acid (TUDCA). It was found that the activity could be maintained with the solution of (3). The activity-maintaining ability of the solution of (8) was comparable to that of (1) Plasma-Lyte A, and that the addition of Imunace does not contribute to the activity for K562. The activity could not be maintained with (6) Klinisalz having a low pH, and (7) a solution containing Klinisalz/MEYLON having an adjusted pH, but having a high osmolarity.
[Fig. 7-2] The high correlation between reduction of viability and the activity after replacement with RPMI was confirmed with good reproducibility.
[Fig. 8] Cytotoxic activity of highly active NK cells after freezing and thawing.
   There were prepared solutions of which pH was adjusted to 7.6 to 8.0 with MEYLON, and which was prepared to have a K⁺ concentration of 5 mEq/L, (1) a solution containing Kenketsu Albumin (Albumin)/K.C.L./physiological saline/MEYLON, and (2) a solution containing Albumin/K.C.L./physiological saline/MEYLON, further, for groups of a solution of which Cl⁻ concentration was further adjusted to 80 mMq/L, (3) a solution containing Albumin/K.C.L./physiological saline/Amizet/water/MEYLON, and (4) a solution containing K.C.L./physiological saline/Amizet/water/MEYLON, as well as (6) Pasma-Lyte A as a positive control. The solutions of (2) and (4) showed good activity-maintaining ability, and the solution of (3) showed a markedly good result.

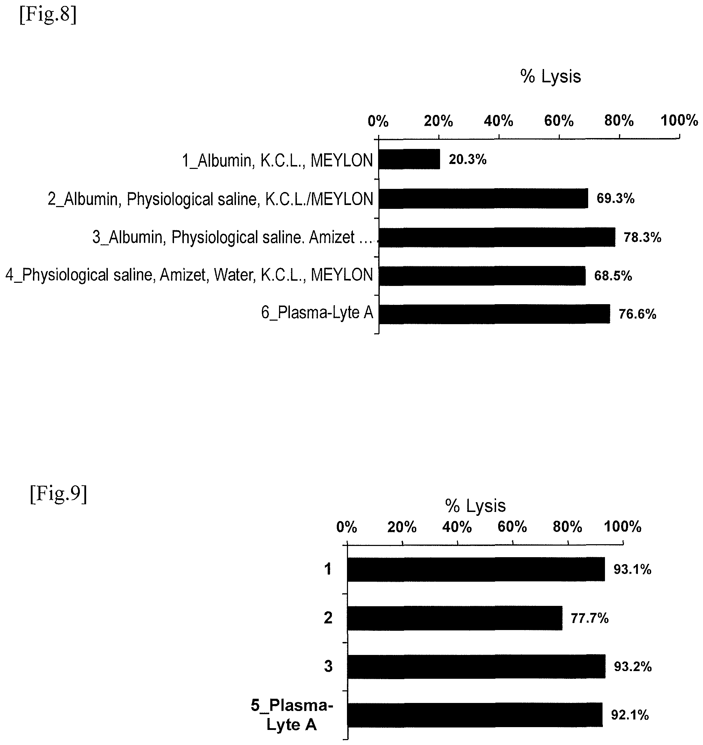

[Fig. 9] Cytotoxic activity of highly active NK cells after freezing and thawing.
   There were prepared (1) a solution containing Albumin/K.C.L./physiological saline/Amizet/water/MEYLON, (2) a solution containing K.C.L./physiological saline/Amizet/water/MEYLON, (3) a solution containing Albumin/K.C.L./physiological saline/Amizet/water/MEYLON, and (5) Pasma-Lyte A as a positive control, all of which were prepared so as to have an osmolarity of 300 mOsm. The solution of (2) showed a good activity-maintaining ability, and the solutions of (1) and (3) showed markedly good results.

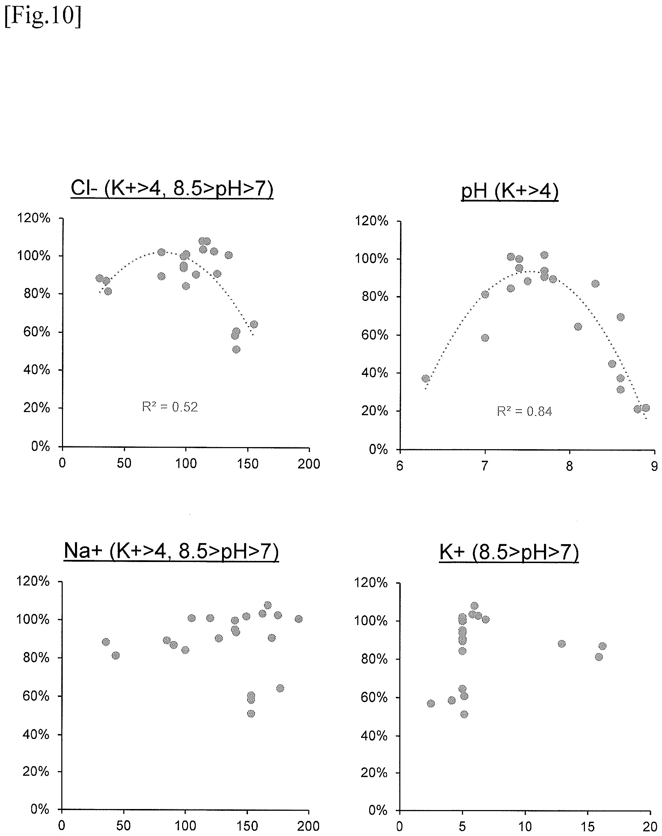

[Fig. 10] Narrowing down of the factors affecting the activity at 3 hours after thawing. The results of the statistical analysis suggested that Cl⁻ concentration and pH have significant influences on the maintenance of the activity, and there may be a threshold value for K⁺.

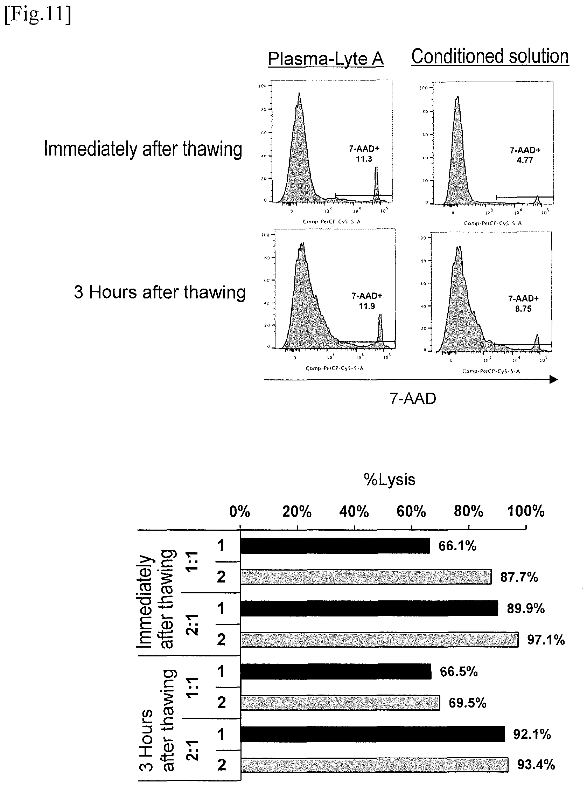

[Fig. 11] Use of another conditioned solution suitable for intravenous administration 1. The upper part shows viabilities based on 7-AAD staining, and the lower part shows cytotoxic activities of (1) Plasma-Lyte A and the conditioned solution (2). K562 cells as the target cells (T) were co-cultured at mixing ratios (E:T) of 1:1 and 2:1 for 2 hours.

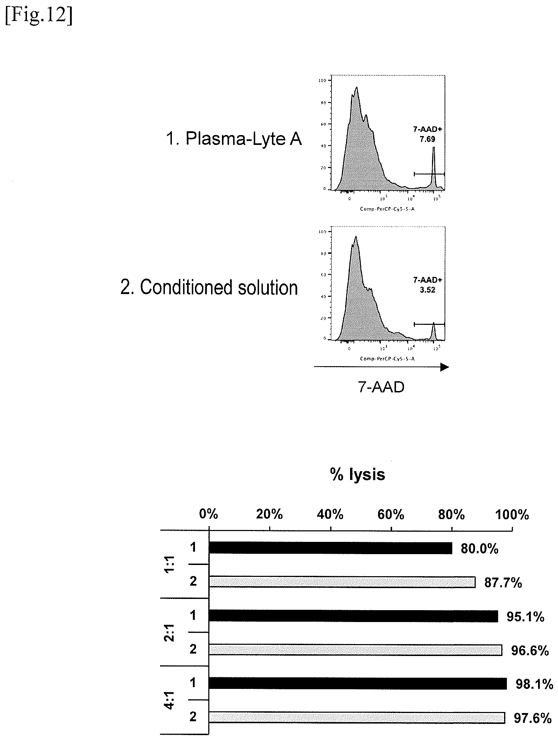

[Fig. 12] Use of another conditioned solution suitable for intravenous administration 2. The upper part shows viabilities based on 7-AAD staining, and the lower part shows cytotoxic activities of (1) Plasma-Lyte A and the conditioned solution (2). K562 cells as the target cells (T) were co-cultured at mixing ratios (E:T) of 1:1, 2:1, and 4:1 for 2 hours.

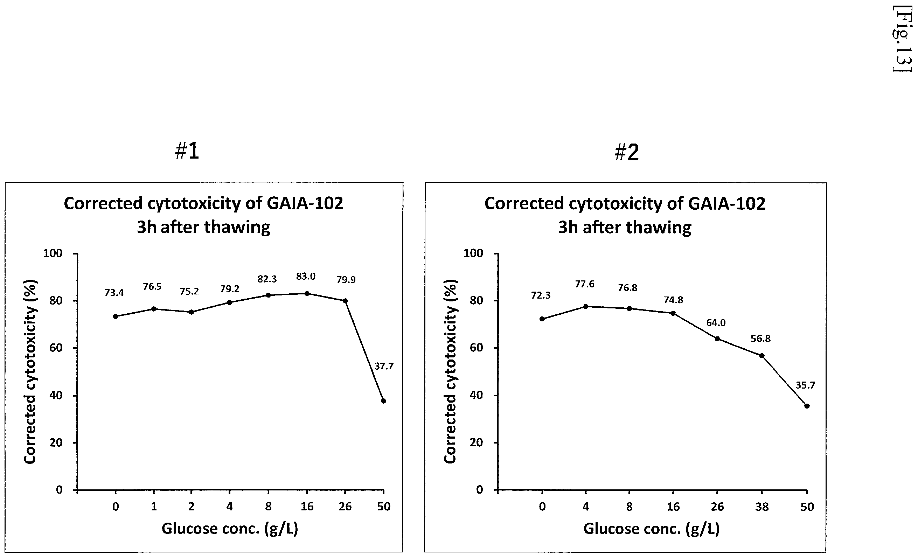

[Fig. 13] Effect of glucose. Cytotoxic activities observed when dilution was performed with Plasma-Lyte A containing various concentrations of glucose. GAIA-102 (E) and K562 cells (T) were mixed at an ET ratio of 0.5:1, 1:1 or 2:1, and cytotoxic activity was measured. A calculation value for E:T=1:1 was obtained by nonlinear regression analysis of the cytotoxic activity ratios calculated for ET ratios at 4 points (including 0) using JMP (registered trademark) Pro statistical analysis software. #1: Glucose concentrations were 0, 1, 2, 4, 8, 16, 26, and 50 g/L, #2: glucose concentrations were 0, 4, 8, 16, 26, 38, and 50 g/L.

### Modes for Carrying out the Invention

In the explanations of the present invention, mM is used with the same meaning as mmol/L, unless especially noted. When a numerical range is expressed as x to y, the range includes the values x and y at both ends.

The present invention relates to a method for thawing or unfreezing frozen cells for administration to humans. For the present invention, thawing or unfreezing means a thawing frozen material, except as otherwise noted. During thawing or unfreezing, a solution may be added to dilute frozen material.

### [Applicable cells]

The present invention can be applied to a variety of cells. One class of the cells to which the present invention can be preferably applied are cells to be administered to humans, preferably cells to be administered to humans that have undergone an activation operation by using some cytokine in vitro, such as NK cells with high cytotoxic activity (highly active NK cells). The activation operation is typically based on incubating the cells with a medium containing interleukin (IL)-2. In the following descriptions, the present invention may be explained by exemplifying cases of applying it to NK cells or highly active NK cells, but those skilled in the art can also appropriately understand the application of the present invention to other types of cells on the basis of such explanations.

In general, NK cells are large granular lymphocytes that are not expressing the T cell receptor (TCR), the universal T cell marker, CD3, and the membrane immunoglobulin, B cell receptor, and they are usually CD16-positive and CD56-positive in humans. Whether or not cells are NK cells can be easily determined by those skilled in the art on the basis of expression patterns of cell surface markers, and so forth. NK cells have cytotoxic activity, and the presence or absence and degree of cytotoxic activity can be measured by various known methods. NK cells can include peripheral blood NK cells, cord blood NK cells, primary NK cells, cultured NK cells, and highly active NK cells.

### (Raw material)

Raw material of highly active NK cells and so forth to which the present invention can be preferably applied may be peripheral blood, cord blood, bone marrow and/or lymph nodes, and blood collected by apheresis method (apheresis blood). The raw material may also be those prepared from at least one kind of cells selected from hematopoietic stem cells derived from any stem cells selected from the group consisting of embryonic stem cells, somatic stem cells, and induced pluripotent stem (iPS) cells, hematopoietic stem cells derived from cord blood, hematopoietic stem cells derived from peripheral blood, hematopoietic stem cells derived from bone marrow blood, cord blood mononuclear cells, and peripheral blood mononuclear cells. The donor of the raw material may be a patient himself/herself who will receive an immunotherapy using highly active NK cells or the like, a close relative of the patient, or a healthy person genetically unrelated to the patient. The donor may consist of a plurality of donors.

### (Culture medium)

Examples of the culture medium used for culturing highly active NK cells and so forth include the KBM501 medium (Kojin Bio, containing 1,750 JRU/mL of IL-2), Cosmedium 008 (Cosmo Bio, containing 1,750 JRU/mL of IL-2), FKCM101 (Fukoku, containing no IL-2 or 175 IU/mL of IL-2), CellGro SCGM medium (CellGenix, Iwai Chemical), X-VIVO15 medium (Lonza, Takara Bio), Gibco (registered trademark) CTS (registered trademark) AIM V (registered trademark) Medium (Thermo Fisher Scientific, serum-free medium of known composition for growth and manipulation of T cells and dendritic cells), CTS OpTmizer T Cell Expansion Basal Medium (Thermo Fisher Scientific, for growth and proliferation of human T lymphocytes), IMDM, MEM, DMEM, RPMI 1640, and so forth, but not limited to these. Preferred examples are the KBM501 medium, FKCM101, and Cosmedium 008. For the present invention, the expression that culture of cells (cells are cultured) means to maintain cells in a medium or a similar solution for a certain period of time for any purpose selected from the group consisting of maintaining cell viability, amplifying cells, and activating cells, unless especially stated. To carry out a treatment at a specific temperature for a certain period of time may be sometimes referred to as incubation (to incubate).

IL-2 may be added to the medium at such a concentration that the purpose of the invention can be achieved. The concentration of IL-2 may range from 2500 to 2813 IU/mL. IL-2 should preferably have a human amino acid sequence thereof, and be produced by a recombinant DNA technique for safety reasons. IL-2 concentration may be expressed in the national standard unit (Japan Reference Unit, JRU) and international unit (IU). 1 IU is approximately 0.622 JRU, and therefore 1,750 JRU/mL of the existing media is equivalent to approximately 2813 IU/mL.

Together with or instead of IL-2 described above, one selected from the group consisting of IL-12, IL-15, and IL-18 may be added at such a concentration that the purpose of the present invention can be achieved (Non-patent document 2, Leong JW et al., Biol. Blood Marrow Transplant, 20 (2014) 463-473). The concentration of each may be 1 pg/mL to 1 µg/mL irrespective of presence or absence or concentration of other cytokines. IL-2 preferably has a human amino acid sequence thereof, and be produced by recombinant DNA technique for safety reasons.

The medium may be supplemented with the subject's autologous serum, human type AB serum available from BioWhittaker and others, or donated blood human serum albumin available from the Japanese Red Cross Society. Autologous serum and human AB serum are preferably added at a concentration of 1 to 10%, and donated blood human serum albumin is preferably added at a concentration of 1 to 10%. Human platelet lysate (HPL) may be added together with or instead of serum. HPL is commercially available, and those of the UltraGRO^{™} series (AventaCell BioMedical), and so forth are commercially available. Sodium heparin may be further added to the medium, when HPL is used.

The medium may contain appropriate proteins, cytokines, antibodies, compounds, and other components, on condition that they do not impair the effectiveness of the NK cell culture. Cytokines may be IL-2, IL-12, IL-15, and IL-18 described above, as well as IL-3, IL-7, IL-21, stem cell factor (SCF), and/or FMS-like tyrosine kinase 3 ligand (Flt3L). All of these should preferably have human amino acid sequences thereof, and be produced by recombinant DNA technique for safety reasons.

The medium should preferably be a serum-free medium. The serum-free medium should preferably contain serum albumin, transferrin, and insulin. Serum-free media for culturing lymphocytes have been developed, and are commercially available, and they can be used for the present invention. One preferred example of serum-free medium is a basic medium supplemented with CTS Immune Cell SR (Thermo Fisher Scientific), which is commercially available as a composition that supports the proliferation of human T cells.

The medium may be replaced or replenished at any time after the start of culture, on condition that the desired culture effect is obtained, but the medium is preferably replaced or replenished every 3 to 5 days.

Culture vessels used for the culture include, but are not limited to, commercially available dishes, flasks, plates, and multi-well plates. Culture conditions are not particularly limited, so long as the culture effect of NK cells is not impaired, but culture conditions of 37°C, 5% CO₂, and saturated water vapor atmosphere are generally used. Culture period is not particularly limited, on condition that the desired culture effect is obtained.

Highly active NK cells, and so forth to which the present invention can be preferably applied include the following [1], [2], [3] and [4].
[1] NK cells having the following characteristics (1) and (2):
   (1) CD16-positivity, high CD56 expression, and CD57-negativity, and
   (2) NKG2C-positivity, NKG2A-negativity to low expression, and CD94-positivity.

The highly active NK cells of [1] may show high CD16 expression. The highly active NK cells of [1] may also have the following characteristics, regardless of whether or not they show high CD 16 expression; (3) Cytotoxic activity of 50% or higher when the NK cells are used as effector cells (E) for K562 cells as target cells (T) in co-culture at a mixing ratio (E:T) of 1:1.

The highly active NK cells of [1] can also be expressed as follows:
NK cells obtained by eliminating CD3-positive cells from peripheral blood mononuclear cells derived from a healthy human using CD3 beads (e.g., CliniMACS CD3, Miltenyi Biotech, catalog no. 130-017-601), LD column (e.g., Miltenyi Biotech, catalog no. 130-042-901), and a separation buffer (e.g., PBS containing 0.5% human AB serum (inactivated), and 2 mM EDTA), and culturing the obtained cell population for 14 days in an appropriate medium (e.g., Cosmedium 008 supplemented with 5% human AB serum (inactivated)), and having the following characteristics (1) and (3):
(1) CD16-positivity, CD56-high expression, and CD57-negativity, and
(3) Cytotoxic activity of 50% or higher when the NK cells are used as effector cells (E) for K562 cells as target cells (T) in co-culture at a mixing ratio (E:T) of 1:1.

For details of the characteristics, and more specific production methods of the highly active NK cells of [1], Japanese Patent Publication (Kokai) No. 2018-193303 can be referred to.

### [2] The following cells:

CCR5-positive, CCR6-positive, CXCR3-positive and CD3-negative cells.

The cells of [2] may also show high CD11c expression.

The cells of [2] can also be expressed as follows
CCR5-positive, CCR6-positive, CXCR3-positive, integrin α1-positive, integrin α3-positive, integrin β3-negative, and CD3-negative cells. Alternatively, CCR5-positive, CCR6-positive, CXCR3-positive, highly CD11a-expressing, highly CD11c-expressing, and CD3-negative cells, of which high expressions are determined by comparison with the expressions in a population of NK cells obtained from peripheral blood that have not been substantially cultured.

According to the studies of the inventors of the present invention, the cells of [2] show extremely high cytotoxic activity against solid tumors forming tumor masses. For details of the characteristics, and more specific production methods of the highly active NK cells of [2], Japanese Patent Publication (Kokai) No. 2019-170176 can be referred to.

### [3] Highly active NK cells obtainable by the following method:

To mononuclear cells obtained from fresh peripheral blood or frozen apheresis blood, add CD3 beads (e.g., CliniMACS CD3, Miltenyi Biotech, 130-017-601 (5 µL per 1 x 10⁷ cells)), and further CD34 beads (e.g., CliniMACS CD34, Miltenyi Biotech, 130-017-501 (2.5 µL per 1 x 10⁷ cells)) in the case of using mononuclear cells obtained from frozen apheresis blood, suspend them, incubate the suspension at 4°C for 15 minutes, then add a separation buffer (e.g., PBS containing 0.5% human type AB serum (inactivated at 56°C for 30 minutes), and 2 mM EDTA), suspend them well, and centrifuge the suspension. Remove the supernatant, and suspend the cells in 0.5 mL of the separation buffer in such a number that the cell number in one LD column (e.g., Miltenyi Biotech, 130-042-901) should be 1 x 10⁸ cells at the maximum. After adding 2 mL of the separation buffer to the LD column beforehand, add the cell suspension to the LD column, and collect eluate from the LD column. Add additional 1 mL of the separation buffer to the LD column, and collect eluate. After centrifuging the collected solution and removing the supernatant, suspend the cells in an appropriate medium (e.g., KBM501 medium containing 5% human AB serum (inactivated at 56°C for 30 minutes) or 5% UltraGRO (AventaCell, HPCPLCRL10) supplemented with 2 U/mL of sodium heparin) at a density of 5 x 10⁵ cells/mL in the case of using fresh peripheral blood, or 1 x 10⁶ cells/mL in the case of using frozen apheresis blood, and culture them up to day 14, with appropriately changing the medium.

For the specific production method of the highly active NK cells of [3], the section of Examples in this description can be referred to.

[4] Cells obtained by obtaining any of the cells of [1] to [3] with adding any selected from the group consisting of IL-12, IL-15, and IL-18 at such a concentration that the purpose of the present invention can be achieved together with or instead of IL-2 at the time of culture. For the specific production method of such cells, Non-patent document 2 mentioned above can be referred to.

In the following explanations, the present invention may be explained with reference to the case of using highly active NK cells as an example, but those skilled in the art can also understand other cases using cells subjected to an in vitro activation operation using some cytokine in accordance with the explanations.

### (Cytotoxic activity)

For the present invention, the term activity or cytotoxic activity used for highly active NK cells, and so forth refers to an ability of subject cells (effector cells, E) to lyse target cells (T), unless especially stated. Cytotoxic activity can be expressed as the percentage of target cells killed by effector cells, and is calculated in accordance with the following equation. (Cell death observed in co-culture with effector cells - Spontaneous cell death (negative control))/(Maximum cell death (positive control) - Spontaneous cell death (negative control)) x 100

When cytotoxic activity is measured, in general, the mixing ratio of the effector cells to the target cells (E:T) and the time of co-culture of effector cells and target cells can be appropriately determined according to the degree of cytotoxic activity of the effector cells, etc., and depending on the types and the intensity of the activity of cells to be used. When NK cells are used as the effector cells, target cells may be, but are not limited to, K562 cells, acute myeloid leukemia cells, and chronic myeloid leukemia cells. The effector cells and target cells, and live cells and dead cells can be distinguished and quantified by using reagents such as antibodies labeled with a radioactive substance, fluorescent dye, or the like. When NK cells are used as the effector cells, the cytotoxic activity can be measured by using K562 cells as the target cells with the conditions of, for example, E:T of 1:0.05 to 10, preferably 1:0.1 to 5, and an incubation time of 0.5 to 18 hours, preferably 1 to 12 hours.

For the present invention, the expression that the activity of NK cells or the like is high means that the cytotoxic activity is 50% or higher when the target cells are K562 cells, and the cells are mixed at E:T of 2:1 and co-cultured for 1 to 3 hours, more specifically 2 hours, unless especially stated. The activity should be preferably 60% or higher, more preferably 70% or higher.

### [Collection]

According to the present invention, prior to the freezing step described later, highly active NK cells or the like to be frozen are collected from the culture system. Collection can be performed by centrifuging the culture to separate the cells from the culture medium. If necessary, EDTA may be added at an appropriate concentration to the culture system to detach adhered cells from the surface of the culture vessel. The surface of the culture vessel may also be washed with an appropriate solution after the cells were detached to further obtain remaining cells. The obtained cells are washed with an appropriate solution and suspended in an appropriate solution, if necessary.

In the collection step, solutions such as culture medium, isotonic solution, and buffer may be used to detach and wash the cells. Examples of usable media include KBM501 medium, Cosmedium 008, FKCM101, CellGro SCGM medium, X-VIVO15 medium, Gibco (registered trademark) CTS (registered trademark) AIM V (registered trademark) Medium, CTS OpTmizer T Cell Expansion Basal Medium, IMDM, MEM, DMEM, and RPMI 1640. Isotonic solution refers to a solution with an osmolarity approximately equal to the osmolarity of body fluid (plasma) (285 ± 5 mOsm/L), and for the present invention, a solution with an osmolarity of 285 ± 13 mOsm/L. For example, the osmolality of Plasma-Lyte A is 294 mOsm/L, and that of PBS (-) is 280 ± 4 mOsm/L (freezing point depression method). Examples of usable isotonic solutions include Plasma-Lyte A (Baxter), saline (physiological saline), Ringer's solution (lactated Ringer's solution, acetated Ringer's solution, hydrogencarbonated Ringer's solution, etc.) and 5% glucose aqueous solution. Examples of buffers that can be used include phosphate-buffered saline (PBS), Tris-hydrochloric acid buffer, Tris-acetic acid buffer, and HEPES buffer.

One of the preferred examples of the solution used in the collection step is a culture medium, more preferably a culture medium for human lymphocytes. The culture medium for human lymphocytes may contain human serum albumin, human transferrin, recombinant human insulin, and recombinant human IL-2. Preferred examples of such a medium are KBM501 medium, FKCM101, and Cosmedium 008. The KBM501 medium contains human serum albumin, human transferrin, recombinant human insulin, and recombinant human IL-2, but no other proteins. The KBM501 medium also contains antibiotics (kanamycin), NaHCO₃, L-glutamine, and pH adjuster.

In the collection step, use of PBS(-) may be undesirable, because it may reduce cell viability after thawing. PBS(-) typically contains 136.9 mM sodium chloride, 2.68 mM potassium chloride, 8.1 mM disodium hydrogenphosphate, and 1.47 mM potassium dihydrogenphosphate.

### [Pretreatment]

For the present invention, prior to the freezing step described later, the highly active NK cells or the like to be frozen may be subjected to a pretreatment. The pretreatment means suspending the collected cells in a solution containing an additive. The pretreatment includes collecting the cells with a solution containing an additive.

The additive used for the pretreatment can be one selected from the group consisting of a bile acid and phenylbutyric acid. Examples of bile acid are tauroursodeoxycholic acid (TUDCA), ursodeoxycholic acid (UDCA), kenodeoxycholic acid, cholic acid, hyodeoxycholic acid, deoxycholic acid, 7-oxolithocholic acid, lithocholic acid, iododeoxycholic acid, iocholic acid, taurokenodeoxycholic acid, taurodeoxycholic acid, glycoursodeoxycholic acid, taurocholic acid, glycocholic acid, and analogues and derivatives thereof. Examples of phenylbutyric acid are 4-phenylbutyric acid (4-PBA), glyceryl tri-(4-PBA), phenylacetic acid, 2-POAA-OMe, 2-POAA-NO₂, 2-NOAA, pharmaceutically acceptable salts, analogues, derivatives and prodrugs thereof. Particularly preferred examples of the additive used for the pretreatment are any selected from the group consisting of TUDCA and 4-PBA.

When a bile acid is used as the additive for the pretreatment, the concentration thereof may be appropriately determined, but it is preferably 100 to 5000 µM, more preferably 200 to 2500 µM, further preferably 400 to 1000 µM. A concentration in such a range is particularly suitable when TUDCA is used. When a phenylbutyric acid is used as the additive for the pretreatment, the concentration thereof may be appropriately determined, but it is preferably 1 to 1000 µM, more preferably 5 to 500 µM, even more preferably 10 to 100 µM. A concentration in such a range is particularly suitable when 4-PBA is used.

Another example of the additive for the pretreatment is dimethyl sulfoxide (DMSO). The concentration thereof may be appropriately determined, but it is preferably 0.5 to 15%, more preferably 1 to 12.5%, further preferably 2 to 10%.

The solution for the pretreatment can be a solution such as medium, isotonic solution, and buffer, like the solution used for the collection. One preferred example of the solution used in the pretreatment is a culture medium, more preferably a medium for culture of human lymphocyte, further preferably KBM501 medium, FKCM101 or Cosmedium 008. The medium used for the pretreatment may also contain human serum albumin, human transferrin, recombinant human insulin, and recombinant human IL-2, and may contain antibiotics (kanamycin), NaHCOs, L-glutamine, and pH adjuster.

The time for the pretreatment is not particularly limited. After the cells are suspended for the pretreatment, the suspension may be allowed to stand for several minutes to several hours, for example, 5 minutes to 4 hours, more preferably 30 minutes to 3 hours. The suspension may be allowed to stand at ambient temperature (e.g., 1 to 30°C, typically 15 to 25°C), and may be allowed to stand in a CO₂ incubator (e.g., 36 to 42°C, typically 37°C).

The cell density during the pretreatment may be appropriately determined, but should be a cell density suitable for cell maintenance. Specifically, the cell density is 1 x 10⁵ to 1 x 10⁷ cells/mL, preferably 2 x 10⁵ to 5 x 10⁶ cells/mL, more preferably 5 x 10⁵ to 2 x 10⁶ cells/mL.

In a particularly preferred embodiment, the pretreatment consists of suspending the cells in the KBM501 medium, FKCM101 or Cosmedium 008 supplemented with 400 to 1000 µM TUDCA or 10 to 100 µM 4-PBA at a cell density of 5 x 10⁵ to 2 x 10⁶ cells/mL. For this treatment, the cells are preferably incubated at 37°C and 5% CO₂ for 30 minutes to 3 hours.

Although the pretreatment is not essential for the present invention, the pretreatment of highly active NK cells or the like with the KBM501 medium supplemented with 4-PBA or TUDCA before freezing can improve the viability (also called recovery rate) after the cells are thawed compared with the case of not performing the pretreatment.

### [Freezing]

According to the present invention, the collected and preferably pretreated cells are frozen by normal procedures. Specifically, the cell count and viability are checked as required, the supernatant is removed by centrifugation, and then the cells are suspended in a cryopreservation solution at an appropriate cell density. After dispensing the cell suspension into cryopreservation containers, it is frozen in a deep freezer at -80°C, and stored. If necessary, it is cryopreserved in a liquid nitrogen tank.

Cryopreservation solutions that can be used in the present invention can contain a sodium salt, potassium salt, sugar, hydrogencarbonate, carbonate, and cryoprotectant.

Sodium salts that can be used are not particularly limited so long as a sodium salt that produces sodium ion when it is dissolved in a solvent is used, and it can be an oxoacid salt, halide, oxide, hydroxide, inorganic salt, organic acid salt, or the like. One kind or a combination of two or more kinds of sodium salts may be used. For the present invention, sodium chloride is preferably used when one kind of salt is used, and sodium chloride and sodium citrate are preferably used when multiple kinds of salts are used. The sodium salt content is not particularly limited, but is preferably 0.01 to 5000 mM, more preferably 0.1 to 1000 mM, further preferably 1 to 300 mM, as the final concentration of total sodium ions contained in the cryopreservation solution.

Potassium salts that can be used are not particularly limited so long as a potassium salt that produces potassium ions when it is dissolved in a solvent is used, and it can be an oxoacid salt, halide, oxide, hydroxide, inorganic salt, organic acid salt, or the like. One kind of potassium salt may be used, or two or more kinds of potassium salts may be used in combination. Potassium chloride is preferably used in the present invention. The potassium salt content is not particularly limited, but as a final concentration of total potassium ions contained in the cryopreservation solution, it is preferably 0.01 to 5000 mM, more preferably 0.1 to 1000 mM, further preferably 1 to 100 mM.

Hydrogencarbonates that can be used are not particularly limited so long as a hydrogencarbonate that produces hydrogencarbonate ions when it is dissolved in a solvent is used, and salts with various cations can be used. Examples include, for example, ammonium hydrogencarbonate, potassium hydrogencarbonate, calcium hydrogencarbonate, sodium hydrogencarbonate, magnesium hydrogencarbonate, and so forth. Carbonates that can be used are not particularly limited so long as a carbonate that produces carbonate ions when it is dissolved in a solvent is used, and salts with a variety of cations can be used. Examples include ammonium carbonate, potassium carbonate, calcium carbonate, sodium carbonate, barium carbonate, magnesium carbonate, and so forth. One kind of these hydrogencarbonates and/or carbonates may be used, or two or more kinds of them may be used in combination. Sodium hydrogencarbonate is preferably used in the present invention. The content of hydrogencarbonate and/or carbonate is not particularly limited, but it is preferably 0.01 to 1000 mM, more preferably 0.1 to 500 mM, further preferably 1 to 100 mM, as the final concentration of total hydrogencarbonate and carbonate ions contained in the cryopreservation solution.

The concentration ratio of sodium ions to potassium ions (sodium ions/potassium ions) in the cryopreservation solution is preferably 1/1000 to 1000/1, more preferably 1/100 to 100/1, further preferably 1/10 to 100/1, further preferably 1/1 to 100/1, further preferably 10/1 to 50/1.

Usable sugars include monosaccharides, oligosaccharides, and sugar alcohols, such as glucose, galactose, fructose, mannose, xylose, arabinose as monosaccharides, trehalose, sucrose, maltose, lactose, cellobiose as oligosaccharides, xylitol, and sorbitol as sugar alcohols. One kind or a combination of two or more kinds of these sugars may be used. For the present invention, the sugar preferably consists of at least one kind of sugar selected from the group consisting of glucose, galactose, fructose, mannose, xylose, and arabinose, more preferably glucose. The content of the sugar in the cryopreservation solution is preferably 0.01 to 100 g/L, more preferably 0.1 to 100 g/L, further preferably 0.25 to 50 g/L.

Examples of usable cryoprotectants include dimethyl sulfoxide (DMSO), hydroxyethyl starch (HES), ethylene glycol, glycerol, and so forth. One kind or a combination of two or more kinds of cryoprotectants may be used. For the present invention, any selected from the group consisting of DMSO and hydroxyethyl starch is preferably used. When DMSO and hydroxyethyl starch are used together as the cryoprotectants, it is preferred that the total content thereof should be in the aforementioned range, and as for the respective concentrations, DMSO concentration is preferably 0.01 to 50%, more preferably 1 to 30%, further preferably 2 to 15%, and hydroxyethyl starch concentration is preferably 0.01 to 50%, more preferably 1 to 30%, further preferably 2 to 15%.

In a preferred embodiment of the present invention, in addition to the essential ingredients of the solution used in the method for cryopreservation of cells described above, the solution may further contain ingredients selected from the group consisting of proteins, magnesium salts and calcium salts. Proteins that can be used include, specifically, serum albumin, serum globulin, and so forth. Serum albumin includes human serum albumin, and bovine serum albumin. For the present invention, human serum albumin is preferred. The protein content in the cryopreservation solution is preferably 0.01 to 50%, more preferably 1 to 30%, further preferably 2 to 15%. Magnesium salts that can be used are not particularly limited so long as a magnesium salt that produces magnesium ions when it is dissolved in a solvent is used, and an oxoacid salt, halide, oxide, hydroxide, inorganic salt, organic acid salt, and so forth can be used. One kind of magnesium salt may be used, or two or more kinds of magnesium salts may be used in combination. Magnesium chloride is preferably used in the present invention. The magnesium salt content of the cryopreservation solution is not particularly limited, but it is preferably 0.01 to 10 mM, more preferably 0.1 to 5 mM, as the final concentration of total magnesium ions in the cryopreservation solution. Calcium salts that can be used are not particularly limited so long as a calcium salt that produces calcium ions when it is dissolved in a solvent is used, and an oxoacid salt, halide, oxide, hydroxide, inorganic salt, organic acid salt, or the like can be used. One kind of calcium salt may be used, or two more kinds of calcium salts may be used in combination. Calcium chloride is preferably used in the present invention. The calcium salt content of the cryopreservation solution is not particularly limited, but is preferably 0.01 to 10 mM, more preferably 0.1 to 5 mM, as the final concentration of total calcium ions in the cryopreservation solution. In addition to the above ingredients, the cryopreservation solution may also contain additional substances that are not injurious to cells, for example, vitamins, amino acids, and so forth. In addition to the above ingredients, the cryopreservation solution may also contain phosphate ions from the viewpoint of pH adjustment and buffering.

The osmolarity of the cryopreservation solution should be within such a range that the cells are not damaged during freezing. The osmolarity is, for example, 500 o 8000 mOsm/L, and may be 1000 to 7500 mOsm/L, 1500 to 7000 mOsm/L, or 1800 to 5000 mOsm/L, from the viewpoints of increasing the penetration of the ingredients into cells during freezing, and inhibition of ice crystal formation. The pH of the cryopreservation solution should be in such a range that the cells are not damaged, for example, 3.0 to 10.0, more preferably 4.5 to 9.0.

For the present invention, commercially available cryopreservation solutions may be used. Examples of products that can be used include the cryopreservation solutions for cells and tissues of the CellBanker series (STEM-CELLBANKER (registered trademark)), more specifically, STEM-CELLBANKER (ZENOAQ, CB045).

The cells are preferably in the logarithmic growth phase at the time of freezing.

The cell density at the time of freezing can be appropriately determined, but it is specifically 1 x 10⁶ to 2 x 10⁸ cells/mL, preferably 2 x 10⁶ to 1 x 10⁸ cells/mL, more preferably 1 x 10⁷ to 5 x 10⁷ cells/mL. In one of the preferred embodiments, the cells are stored at a cell density of 4 x 10⁷ cells/mL in a 5-mL volume container. The characteristic of the highly active NK cells that they can be frozen at a high density at the time of freezing for shipping allows to make the product more compact, and contributes to lower shipping costs.

### [Thawing or unfreezing, and dilution]

In the present invention, cryopreserved cells can be thawed by various procedures. For example, such cells can be quickly thawed by incubating the cryopreservation container containing the cells in a warm bath at 37°C or the like, with shaking the container as required. Alternatively, cryopreserved cells can be spontaneously thawed by leaving them at room temperature without any active heating after they are taken out from the freezer. At the time of or after thawing, the thawing cell cryoproduct can be diluted with an appropriate solution.

### (Liquid used for dilution)

The present invention provides a solution for suspending cells for administration to humans, more specifically, a solution for diluting a cell cryoproduct, which satisfies the following criteria:
(1) containing potassium ion,
(2) having a pH of 6.4 or higher,
(3) not containing chloride ions at a concentration of 135 mEq/L or higher (i.e., the concentration of chloride ions is lower than 135 mEq/L or the solution does not contain chloride ions),
(4) not containing glucose at a concentration of 5.55 mM or higher (i.e., the concentration of glucose is lower than 5.55 mM or the solution does not contain glucose),
(5) not containing calcium ions at a concentration of 0.423 mM or higher (i.e., the concentration of calcium ions is lower than 0.423 mM or the solution does not contain calcium ions), and
(6) having an osmolarity of 200 to 396 mOsm.

For the present invention, the expression that a solution does not contain a certain ingredient means that the ingredient is not detected by ordinary measurement methods, unless otherwise stated.

The concentration of potassium ions in the solution for dilution is preferably 0.50 to 8.0 mM, more preferably 1.0 to 7.0 mM, further preferably 2.5 to 6.0 mM. Alternatively, it is preferred that the solution contains 0.496 to 5.96 mM potassium chloride.

In one preferred embodiment, the concentration of potassium ions in the solution for dilution is 4.0 mEq/L or higher. The maximum concentration is not particularly limited, but is, for example, 18.0 mEq/L or lower, preferably 15.0 mEq/L or lower, more preferably 10.0 mEq/L or lower, further preferably 7.50 mEq/L or lower.

In another preferred embodiment, the solution for dilution does not contain calcium ions, irrespective of the presence or absence of the other ingredients and concentrations thereof. In another preferred embodiment, the solution for dilution does not contain glucose, irrespective of the presence or absence of the other ingredients and concentrations thereof. In further another preferred embodiment, pH of the solution for dilution is 7.0 to 8.3, preferably 7.2 to 8.1, more preferably 7.3 to 7.9.

The ions contained in the solution for dilution may be derived from salts that can be used as pharmaceuticals. Such salts may be carbonates, hydrogencarbonates, oxoates, halides, oxides, hydroxides, inorganic salts or organic acid salts. One type of salt or a combination of two or more types of salts may be used.

The solution for dilution may contain a sodium salt, gluconate, and acetate. It may also contain a magnesium salt. The concentration of sodium ions in the solution for dilution is preferably 14.0 to 200 mM, more preferably 28.0 to 182 mM, further preferably 70.0 to 168 mM. Or the solution for dilution preferably contains 9.00 to 108 mM sodium chloride, 2.0 to 27.7 mM sodium gluconate, and 2.7 to 32.5 mM sodium acetate. The concentration of gluconate ions in the solution for dilution is preferably 2.3 to 32.3 mM, more preferably 4.6 to 29.9 mM, further preferably 12.5 to 27.7 mM. The concentration of acetate ions in the solution for dilution is preferably 2.7 to 37.8 mM, more preferably 5.4 to 35.1 mM, further preferably 13.5 to 32.5 mM.

The solution for dilution can be constituted by approved pharmaceuticals. Examples of such pharmaceuticals are listed below.

### Generic name: Amizet

Bland name: Amizet B Infusion
Therapeutic classification: Comprehensive amino acid preparation
Composition (in 1 bag/200 mL)

Active ingredients: L-Isoleucine 1,700 mg, L-leucine 2,700 mg, lysine malate 2,432 mg (1,600 mg as L-lysine), L-methionine 780 mg, L-phenylalanine 1,540 mg, L-threonine 960 mg, L-tryptophan 320 mg, L-valine 1,800 mg, cysteine malate 310 mg (200 mg as L-cysteine), L-tyrosine 100 mg, L-arginine 2,220 mg, L-histidine 940 mg, L-alanine 1,720 mg, L-aspartic acid 100 mg, L-glutamic acid 100 mg, glycine 1,100 mg, L-proline 1,280 mg, and L-serine 840 mg
Additive: Succinic acid (pH adjuster) appropriate amount
Electrolyte: Na⁺ and Cl⁻ are not contained.

### Generic name: MEYLON

Bland name: MEYLON Injection 8.4%
Common name: Sodium hydrogencarbonate
Composition (in 20mL)
   Sodium hydrogencarbonate 1.68 g (8.4%)
   Electrolyte concentration: Na⁺ 1000 mEq/L, HCO₃⁻ 1000 mEq/L

### Generic name: K.C.L.

Bland name: K.C.L. Drip Injection 15%
Common name: Potassium chloride
Formulation: Potassium chloride formulation
Composition
   Ingredients: Potassium chloride 3 g (15 w/v%, 2 M solution) [potassium (K) content 40 mEq (1573.36 mg)]
   Additive: sodium riboflavin phosphate (riboflavin phosphate sodium) 6 mg

### Generic name: Albumin (Kenketsu Albumin)

Brand name: Albumin (Kenketsu Albumin) 25% I.V. 12.5g/50mL
Therapeutic classification: Plasma fractionation product (human serum albumin preparation)
Composition (in 50mL)
   Active ingredient: human serum albumin 12.5 g
   Additives: acetyltryptophan 250.97 mg, sodium hydroxide 43.44 mg, sodium caprylate 169.75 mg
   pH 6.4 to 7.4, osmotic ratio (ratio to physiological saline) 0.5 to 1.0

### (Preferred embodiments)

One particularly preferred embodiment of the solution for dilution is the followings.

| | |
|---|---|
| Albumin (Kenketsu Albumin) 25% | 20.0 mL |
| Physiological saline | 111.12 mL |
| Amizet | 19.08 mL |
| Distilled water | 56.68 mL |
| MEYLON | 1.6 mL |
| K.C.L. | 0.4 mL |
| pH=7.2 | |

Another preferred composition consists of the followings.

| | |
|---|---|
| Albumin (Kenketsu Albumin) 25% | 11.112 mL |
| Physiological saline | 111.12 mL |
| Amizet | 19.08 mL |
| Water | 56.68 mL |
| MEYLON | 1.6 mL |
| K.C.L. | 0.4 mL |
| pH=7.2 | |

Another preferred composition consists of the followings.

| | |
|---|---|
| Albumin (Kenketsu Albumin) 25% | 20.0 mL |
| Physiological saline | 111.12 mL |
| Amizet | 19.08 mL |
| Water | 56.68 mL |
| MEYLON Injection | 1.6 mL |
| K.C.L. | 0.4 mL |
| pH=7.2 | |

Cells suspended along with the solution for cryopreservation in the solution for dilution can be used as they are for administration. From the viewpoint of use for administration, it is preferred that the mixture of the cryopreservation solution and the solution for dilution should be isotonic (i.e., it has an osmolarity approximately equal to that of body fluid, specifically 285 ± 13 mOsm/L). Since the solution for cryopreservation is a hypertonic solution (e.g., 1500 to 7000 mOsm/L) in a preferred embodiment, the solution for dilution may be a low osmotic solution. Those skilled in the art can appropriately determine concentrations of the ingredients of the solution for dilution in consideration of the magnitude of dilution of the solution for cryopreservation with the solution for dilution.

In any composition of the solution for dilution, it is preferred that the solution for dilution does not contain serum at a concentration of 40% or higher, and it is more preferred that it does not contain serum at all. This is because if the solution for dilution contains serum, cell viability may be reduced.

Although the density of the cells suspended in the solution for dilution can be optionally chosen, it may be a cell density suitable for maintenance of the cells or cell density suitable for administration. Specifically, it is 1 x 10⁵ to 1 x 10⁷ cells/mL, preferably 2 x 10⁵ to 5 x 10⁶ cells/mL, more preferably 5 x 10⁵ to 2 x 10⁶ cells/mL.

The cells can be maintained in the solution for dilution for a relatively long period of time. After suspending the cells in the solution for dilution, the suspension may be allowed to stand for several minutes to several hours, e.g., 5 minutes to 6 hours, more preferably 30 minutes to 4 hours. They may be allowed to stand at ambient temperature (e.g., 1 to 30°C, typically 15 to 25°C), or in a CO₂ incubator (e.g., 36 to 42°C, typically 37°C).

### [Use in pharmaceutical compositions]

The present invention provides a pharmaceutical composition containing highly active NK cells, which have been collected by an appropriate method, pretreated as required, and cryopreserved, and so forth.

The pharmaceutical composition provided by the present invention can be used for the treatment and/or prevention of various diseases susceptible to highly active NK cells, and so forth. Examples of such diseases are cancers and infectious diseases, and specifically, they include, but not limited to, skin cancer, oral cancer, gallbladder cancer, bile duct cancer, lung cancer, liver cancer, stomach cancer, colon cancer, pancreatic cancer, kidney cancer, ovarian cancer, bladder cancer, prostate cancer, neuroblastoma, leukemia, and infectious diseases caused by viruses, bacteria or the like. The inventors of the present invention confirmed the effect of use of cells frozen and thawed by the method of the present invention on animal models of colon cancer, which should otherwise die within 30 days without any treatment.

A cell therapy using the pharmaceutical composition of the present invention may be performed solely, or in combination with surgical therapy, chemotherapy, radiation therapy, antibody drugs, and so forth.

The characteristics of one embodiment of the pharmaceutical composition provided by the present invention are shown below.

(Dosage form)
   Injection (cell suspension)
(Ingredient and content)
   Component cells: Highly active NK cells, etc.
   Content: 6 x 10⁶ to 4.8 x 10⁹ cells/60 kg
(Sub-ingredients)
   Complex electrolyte solution 10 to 45%
   Sodium chloride solution 10 to 45%
   20 to 30% Human serum albumin solution 5 to 30%
   Dimethyl sulfoxide 2 to 15%
   Others
      or
   Cryopreservation solution acceptable as a pharmaceutical additive 100%

### (Preparation method)

The frozen composition is thawed in a thermostatic bath at 37°C or the like until it is completely thawed. Immediately after the thawing, the cells are aseptically suspended in a separately prepared isotonic solution acceptable as a pharmaceutical additive.

### (Stability after thawing)

The shelf life of the composition after thawing is 6 hours, preferably 4 hours, when it is stored at room temperature.

### Examples

### 1. Methods commonly used in Examples

### A) Method for culturing highly active NK cells GAIA-102

Frozen apheresis blood (HemaCare/Cellero) as the raw material was thawed, and then the cells were washed and concentrated by using Lovo Cell Processing System (Fresenius Kabi) to obtain PBMCs.

CD3-Positive and CD34-positive cells were removed from the resulting PBMCs by using CliniMACS Prodigy (registered trademark, Miltenyi Biotec), and the cells were eluted with KBM501 medium*¹. The numbers of the cells in the eluate was counted, and the total cell count was calculated. The culture was performed by using an adhesion culture bag 640 cm², to which 200 mL per bag of a cell suspension prepared in the KBM501 medium at a density of 5 x 10⁵ cells/mL was inoculated, in a CO₂ incubator (37°C, 5% CO₂). On day 9 of the culture, the KBM501 medium was further added to make the final volume 650 mL per bag, and the incubation was continued until day 14.

*1: KBM501 (Kohjin Bio) supplemented with 5% UltraGRO (AventaCell, HPCPLCRL10) and 2 U/mL heparin sodium (Nipro).

### B) Method for collecting highly active NK cells GAIA-102

On day 14 of the culture, the culture medium was collected, and 1 mM EDTA was added to the culture bag to detach the adhered cells. The total volume of the collected culture medium and the detached cells were centrifuged, and the cells were washed and resuspended in the KBM501 medium.

### C) Method for freezing highly active NK cells GAIA-102

The number of viable cells of GAIA-102 obtained by the procedures described as the methods for culturing and collecting highly active NK cells was counted, and 2 x 10⁸ cells were suspended in 5 mL of HSC-BANKER (ZENOAQ, CB071) and frozen at -80°C.

### D) Method for measuring viability based on 7-AAD staining

The GAIA-102 cells thawed under each condition were prepared on a 96-well plate (IWAKI, 4870-800SP) at a density of 1 x 10⁵ cells/well, and centrifuged, the supernatant was removed, then a 7-AAD solution (Beckman Coulter, A07704) diluted with PBS was added to suspend the cells, and the plate was incubated at room temperature for 20 minutes. After the staining, measurement of the cells was performed by using a flow cytometer (BD LSR Fortessa, BD Biosciences), the results were analyzed with FlowJo software, and the viability was calculated from the 7-AAD-positive rate.

### E) Method for measuring cytotoxic activity against tumor cells

For the measurement of cytotoxic activity, a group of the NK cells reacted with the K562 cells, a group solely consisting of the K562 cells as a negative control, and a group of the K562 cells fixed with 10% formalin as a positive control were prepared.

### <<GAIA-102>>

The GAIA-102 cells were thawed and diluted under each condition, and a necessary amount of the cells were taken on the basis of the number of viable cells at the time of freezing, and then prepared at a density of 1 × 10⁶ cells/ mL in 10% FBS/RPMI 1640.

### «K562»

The K562 cells were suspended in serum-free RPMI 1640 medium, stained with PKH26 Red Fluorescent Cell Linker Kit, and then prepared at a density of 2 x 10⁶ cells/mL in 10% FBS/RPMI 1640.

The GAIA-102 cells and K562 cells were added and mixed in wells of 96-well plate (IWAKI, 4870-800SP) at a cell ratio of 2:1, and allowed to react at 37°C for 2 hours under 5% CO₂. After the reaction, the plate was centrifuged (500 x g, 5 minutes), the supernatant was removed, then a 7-AAD solution diluted with PBS was added to suspend the cells, and the suspension was incubated at room temperature for 20 minutes. Measurement was performed by using a flow cytometer, and the results were analyzed with the FlowJo software to calculate the cytotoxic activity rate (% Lysis)*². 2*: Cytotoxic activity rate = (K562 Cell dead cell rate - Negative control dead cell rate)/(Positive control dead cell rate - Negative control dead cell rate) x 100

### Reagents and infusion agents used

The following reagents and infusion agents were used in the verification of solutions for thawing.

Sodium chloride (Nacalai Tesque)
Potassium chloride (Nacalai Tesque)
Magnesium chloride hexahydrate (Nacalai Tesque)
Sodium acetate trihydrate (Nacalai Tesque)
Sodium gluconate (Nacalai Tesque)
TUDCA (Tokyo Kasei Kogyo Co., Ltd.)
Water (Nacalai Tesque)
Plasma-Lyte A (Baxter)
Physiological saline, Otsuka Normal Saline Injection (Otsuka Pharmaceutical Co., Ltd.)
D-PBS (Nakalai Tesque)
SOLMALT Infusion (Terumo Corporation)
THAM Injection Set (Otsuka Pharmaceutical Co., Ltd.)
MEYLON Injection 8.4% (Otsuka Pharmaceutical Co., Ltd.)
Klinisalz Infusion (Kyowa Criticare Co., Ltd.)
Imunace Injection 35 (Kyowa Pharmaceutical Co., Ltd.)
Albumin (Kenketsu Albumin) 25% I.V. Injection (Japan Pharmaceutical Co., Ltd.) K.C.L. Drip Injection 15% (Maruishi Pharmaceutical Co., Ltd.)
Amizet B Infusion (Terumo Corporation)

### 2. Methods for specific examples

### 2-1) Verification of Plasma-Lyte A ingredients

The GAIA-102 cells frozen for 48 hours or longer were thawed on a water bath at 37°C and then allowed to stand at room temperature for up to 3.5 hours. Viability measurement based on 7-AAD staining and cytotoxic activity measurement were performed 30 minutes and 3 hours after the thawing.

After the thawing, the cells were diluted 41-fold with the solutions (1) to (6) shown in the following table. There were set a group of (1) a solution having a composition based on the concentrations of the ingredients of Plasma-Lyte A, but not containing sodium gluconate, a group of (2) a solution having a composition based on the concentrations of the ingredients of Plasma-Lyte A, but not containing sodium gluconate, and containing the other ingredients increased by 16% each to adjust osmolarity, a group of (3) a solution having a composition based on the concentrations of the ingredients of Plasma-Lyte A, but not containing sodium gluconate and having an osmolarity adjusted with NaCl, a group of (4) Plasma-Lyte A as a positive control, a group of (5) physiological saline, and a group of (6) physiological saline containing sodium gluconate at the same concentration as that of Plasma-Lyte A. The osmolarities were obtained by calculation for all the cases unless especially noted.

**[Table 1]**

| | 1 | 2 | \| 3 | 4 | 5 | 6 |
|---|---|---|---|---|---|---|
| | Solution of the same ingredients as Plasma-Lyte A, but not containing sodium gluconate | | | Plasma-Lyte A | Physiological saline | Physiological saline + Na gluconate |
| | - | Osmolarity adjustment 16% | Osmolarity adjustment NaCl | | | |
| NaCl | 2.63 g | 3.05 g | 3.255 g | (2.63 g) | (4.5 g) | |
| KC1 | 0.185 g | 0.215 g | 0.185 g | (0.185 g) | - | - |
| MgCl₂·6H₂O | 0.150 g | 0.174 g | 0.150 g | (0.150 g) | - | - |
| CH₃COONa·3H₂O | 1.840 g | 2.134 g | 1.840 g | (1.840 g) | - | - |
| Na gluconate | - | - | - | (2.9 g) | - | 2.9 g |
| Water (ml) | Up to 500 | Up to 500 | Up to 500 | - | - | 80.65 |
| Plasma-Lyte A (ml) | - | - | - | 500 | - | - |
| Physiological saline (ml) | - | - | - | - | 500 | Up to 500 |

| | | | | | | |
|---|---|---|---|---|---|---|
| *The parenthesized numbers are contents in the formulations. | | | | | | |

### Results:

No decrease in viability was observed after the thawing for all the groups. However, a marked decrease in the activity was observed in the groups of (5) physiological saline, and (6) physiological saline to which sodium gluconate was added at the same concentration of as Plasma-Lyte A (Fig. 1). This phenomenon was observed at 3 hours after the thawing and dilution and was not observed immediately after the thawing. It became clear that it is impossible to maintain the activity after thawing and dilution using only NaCl and sodium gluconate.

### 2-2) Identification of essential ingredients of Plasma-Lyte A

Verification was performed for the ingredients other than sodium gluconate. The GAIA-102 cells frozen for 48 hours or longer were thawed on a water bath at 37°C and then allowed to stand at room temperature up to 3.5 hours. Viability measurement based on 7-AAD staining and cytotoxic activity measurement were performed 30 minutes and 3.5 hours after the thawing.

After the thawing, the cells were diluted 41-fold with the solutions shown in the following table. There were set groups of (1) to (3) solutions having a composition based on the concentrations of the ingredients of Plasma-Lyte A, but not containing sodium gluconate, from which each of Mg²⁺, K⁺, and CH₃COO⁻ (acetate) was excluded, respectively, and groups of (4) to (6) physiological saline supplemented with each of the ingredients, respectively.

**[Table 2]**

| | 1 | 2 | 3 | 4 | 5 | 6 | 7 |
|---|---|---|---|---|---|---|---|
| | Mg²⁺ -free | K⁺ -free | Acetate--free | Physiological saline + Mg²⁺ | Physiological saline + K⁺ | Physiological saline + acetate⁻ | Plasma-Lyte A |
| NaCl | 2.630 g | 2.630 g | 2.630 g | - | - | - | - |
| KC1 | 0.185 g | - | 0.185 g | - | 0.185 g | - | - |
| MgCl₂·6H₂O | - | 0.150 g | 0.150 g | 0.150 g | - | - | - |
| CH₃COONa·3H₂O | 1.840 g | 1.840 g | - | - | - | 1.840 g | - |
| Water (ml) | Up to 500 | Up to 500 | Up to 500 | - | - | - | - |
| Plasma-Lyte A (ml) | - | - | - | - | - | - | 500 |
| Physiological saline (ml) | - | - | - | Up to 500 | Up to 500 | Up to 500 | - |

### Results:

In terms of viability, a slight decrease was observed only in the group of K⁺- free (2) at 3 hours after the thawing and dilution. On the other hand, decrease in the activity was observed in groups of (2) and (3) wherein K⁺ and CH₃COONa were excluded, respectively, but not in the group (1) in which Mg+ was removed. Further, the experiments of the groups (4) to (6), in which one of the ingredients was added to physiological saline, respectively, showed that only K⁺ was an ingredient that contributes to the activity all by itself (Fig. 2).

### 2-3) Search for optimal concentration ranges of K⁺ and CH₃COO⁻

The GAIA-102 cells frozen for 48 hours or longer were thawed on a water bath at 37°C and then allowed to stand at room temperature for up to 3.5 hours. Viability measurement based on 7-AAD staining and cytotoxic activity measurement were performed 30 minutes and 3.5 hours after the thawing.

After the thawing, the cells were diluted 41-fold with the solutions shown in the following table. There were prepared (1) Plasma-Lyte A as a positive control, (2) physiological saline as a negative control, and (3) to (6) solutions having a composition based on the concentrations of the ingredients of Plasma-Lyte A, but not containing sodium gluconate and magnesium chloride, and having osmolarity ratios to physiological saline of 0.80, 0.95, 1.0, and 1.10, respectively. There was also set a group of (7) a solution having a composition based on the concentrations of the ingredients of Plasma-Lyte A, of which osmolarity ratio was adjusted to 1.0 with the content of NaCl alone.

**[Table 3]**

| | 1 | 2 | 3 | 4 | 5 | 6 | 7 |
|---|---|---|---|---|---|---|---|
| | Plasma-Lyte A | Physiological saline | Na gluconate and MgCl₂-free solutions containing the same ingredients as Plasma-Lyte A | | | | |
| | Osmolarity ratio 0.95 | Osmolarity ratio 1.00 | Osmolarity ratio 0.80 | Osmolarity ratio 0.95 | Osmolarity ratio 1.00 | Osmolarity ratio 1.10 | Osmolarity ratio 1.00 |
| NaCl | - | | 2.630 g | 3.130 g | 3.290 g | 3.600 g | 3.510 g |
| KCl | - | - | 0.185 g | 0.220 g | 0.231 g | 0.253 g | 0.185 g |
| CH₃COONa·3H₂O | - | - | 1.840 g | 2.190 g | 2.300 g | 2.520 g | 1.840 g |
| Water | - | - | 500 mL | 500 mL | 500 mL | 500 mL | 500 mL |
| Plasma-Lyte A | 500 mL | - | - | - | - | - | - |
| Physiological saline | - | 500 mL | - | - | - | - | - |

### Results:

No decrease in viability was observed for all the concentrations up to 3.5 hours after the thawing and dilution. No effect on the activity was observed for the groups (3) to (6), but a decreasing tendency was observed for the group (7) in which the osmolarity ratio was adjusted to 1.0 with the content of NaCl alone (Fig. 3).

### 2-4) Verification of substitutability of ready-made products for CH₃COONa/KCl 1

The GAIA-102 cells frozen for 48 hours or longer were thawed on a water bath at 37°C and then allowed to stand at room temperature for up to 3 hours. Cytotoxicity activity was measured 3 hours after the thawing.

After the thawing, the cells were diluted 41-fold with the solutions shown in the following table. There were set groups of (1) Plasma-Lyte A as a positive control, and (2) physiological saline as a negative control, and (3) phosphate buffer supplemented with K⁺ for a group of phosphate buffer having a K⁺ concentration of 5 mEq/L, (4) THAM Injection, (6) a solution containing physiological saline/MEYLON/KCl, and (7) a solution containing water/MEYLON/KCl/NaCl were prepared. In addition, there were also prepared (5) a solution containing THAM/MEYLON prepared so as to have a K⁺ concentration of 2.5 mEq/L, and (8) a solution containing water/CH₃COONa/KCl/NaCl.

**[Table 4]**

| | 1 | 2 | 3 | 4 | 5 | 6 | 7 | 8 |
|---|---|---|---|---|---|---|---|---|
| | Plasma-Lyte A | Physiological saline | PBS KCl | THAM | THAM Physiological saline | Physiological saline MEYLON KCl | Water MEYLON KCl NaCl | Water Na acetate KCl NaCl |
| Plasma-Lyte A | 500 mL | - | - | - | - | - | - | - |
| Physiological saline | - | 500 mL | - | - | - | 500 mL | - | - |
| PBS | - | - | 500 mL | - | - | - | - | - |
| THAM | - | - | - | 500 mL | 250 mL | - | - | - |
| MEYLON | - | - | - | - | 250 mL | 13.5 mL | 13.5 mL | - |
| Water | - | - | - | - | - | - | 500 mL | 500 mL |
| KCl | - | - | 0.037 g | - | - | 0.185 g | 0.185 g | 0.090 g |
| NaCl | - | - | - | - | - | - | 4.5 g | 2.630 g |
| CH₃COONa·3H₂O | - | - | - | - | - | - | - | 1.840 g |

### Results:

For the group of (3) phosphate buffer supplemented with K⁺ to achieve a K⁺ concentration of 5 mEq/L, an increase in the activity was observed compared with (2) physiological saline, but it could not satisfactorily maintain the activity. The maintenance of the activity was also insufficient with (5) the solution containing THAM/MEYLON prepared so as to have a K⁺ concentration of 2.5 mEq/L and (8) the solution containing water/CH₃COONa/KCl/NaCl, (Fig. 4).

### 2-5) Verification of substitutability of ready-made products for CH₃COONa/KCl 2

The GAIA-102 cells frozen for 48 hours or longer were thawed on a water bath at 37°C and then allowed to stand at room temperature for up to 3 hours. Cytotoxicity activity was measured 3 hours after the thawing.

After the thawing, the cells were diluted 41-fold with the solutions shown in the following table. There were set groups of (1) Plasma-Lyte A as a positive control, and (2) physiological saline as a negative control, and there were prepared (7) SOLMALT (SOLMALT Infusion), as well as (3) a solution containing SOLMALT/MEYLON, and (4) a solution containing SOLMALT/THAM, for groups of solutions based on SOLMALT of which pH is adjusted. In addition, there were also prepared (5) a solution having the same composition as Plasma-Lyte A except that sodium gluconate and Mg²⁺ were both excluded, in which the concentrations of the other ingredients were the same as those in Plasma-Lyte A, and (6) a solution having the same composition as Plasma-Lyte A except that sodium gluconate and Mg²⁺ were both excluded, and only NaCl was contained in a decreased amount so that the solution had the same osmolarity as the solution of (5). Further, there was additionally set a group of (8) SOLMALT having a pH adjusted with THAM, and an osmolarity reduced by adding water after the pH adjustment. The pH values of the prepared solutions were also measured.

**[Table 5]**

| | 1 | 2 | 3 | 4 | 5 | 6 | 7 | 8 |
|---|---|---|---|---|---|---|---|---|
| | Plasma-Lyte A | Physiological saline | SOLMALT MEYLON | SOLMALT THAM | Water Na acetate KCl NaCl | Water Na acetate KCl NaCl | SOLMALT | SOLMALT THAM Water |
| Plasma-Lyte A | 500 mL | - | - | - | - | - | - | - |
| Physiological saline | - | 500 mL | - | - | - | - | - | - |
| SOLMALT | - | - | 500 mL | 500 mL | - | - | 500 mL | 500 mL |
| THAM | - | - | - | 50 mL | - | - | - | 50 mL |
| MEYLON | - | - | 25 mL | - | - | - | - | - |
| Water | - | - | - | - | 500 mL | 500 mL | - | 126.5 mL |
| KCl | - | - | - | - | 0.180 g | 0.230 g | - | - |
| NaCl | - | - | - | - | 2.630 g | 2.400 g | - | - |
| CH₃COONa·3H₂O | - | - | - | - | 1.840 g | 2.300 g | - | - |

### Results:

Although the activity could not be maintained with (7) SOLMALT, an activity-improving tendency was observed for (3) the solution containing SOLMALT/MEYLON, and (4) the solution containing SOLMALT/THAM, which were based on SOLMALT, and of which pH values were adjusted. This tendency was confirmed with reproducibility, and similar results were obtained for the group of (8) SOLMALT having a pH adjusted with THAM, and an osmolarity reduced by adding water after the pH adjustment. From these results, it was also experimentally confirmed that it becomes difficult to maintain the activity when the pH decreases to 6.0, and acceptable results can be obtained up to pH 8.8 (Fig. 5).

### 2-6) Relationship between maintenance of activity and viability

For the groups for which dilution was performed with the solutions described in 2-5), viability measurement based on 7-AAD staining and cytotoxic activity measurement were performed 1 and 3 hours after the thawing, and viability of the GAIA-102 cells was also analyzed after the reaction for the cytotoxic activity measurement.

### Results:

It was found that decrease in viability, which was not observed immediately after the thawing and dilution, was observed after replacement with RPMI. After the replacement with RPMI, decrease in viability was observed over time, and it was found that the extent of the decrease was highly correlated with the activity (Fig. 6).

### 2-7) Relationship between maintenance of activity and osmolarity/pH

The GAIA-102 cells frozen for 48 hours or longer were thawed on a water bath at 37°C and then allowed to stand at room temperature up to 3 hours. Cytotoxicity activity was measured 3 hours after the thawing. After the thawing, the cells were diluted 41-fold with the solutions shown in the following table. There were set groups of (1) Plasma-Lyte A as a positive control, and (2) physiological saline as a negative control, and there were prepared (3) a solution consisting of SOLMALT and MEYLON at a ratio of 20:1, (4) a solution consisting of SOLMALT and MEYLON at a ratio of 10:1, and (5) a solution containing SOLMALT/MEYLON/water for groups of solutions prepared based on SOLMALT with pH adjustment with MEYLON, as well as (6) Klinisalz, and (7) a solution containing Klinisalz/MEYLON for a group of a solution prepared based on Klinisalz with pH adjustment with MEYLON. There were also prepared (8) a solution containing Plasma-Lyte A + 140,000 IU/mL Imunace and (9) a solution containing Plasma-Lyte A + 875 µM tauroursodeoxycholic acid (TUDCA). The pH values of the prepared solutions were also measured.

**[Table 6]**

| | 1 | 2 | 3 | 4 | 5 | 6 | 7 | 8 | 9 |
|---|---|---|---|---|---|---|---|---|---|
| | Plasma-Lyte A | Physiological saline | SOLMALT MEYLON (20:1) | SOLMALT MEYLON (10:1) | SOLMALT MEYLON Water | Klinisalz | Klinisalz MEYLON | Plasma-Lyte A Imunace | Plasma-Lyte A Imunace TUDCA |
| Plasma-Lyte A | 500 mL | - | - | - | - | - | - | 500 mL | 500 mL |
| Physiological saline | - | 500 mL | - | - | - | - | - | - | - |
| SOLMALT | - | - | 500 mL | 500 mL | 375 mL | - | - | - | - |
| MEYLON | - | - | 25 mL | 50 mL | 37.5 mL | - | 20 mL | - | - |
| Water | - | - | - | - | 125 mL | - | - | - | - |
| Klinisalz | - | - | - | - | - | 500 mL | 500 mL | - | - |
| Imunace | - | - | - | - | - | - | - | 5 mL | - |
| 100 mM TUDCA | - | - | - | - | - | - | - | - | 4.375 mL |

For the groups in which the cells were thawed with the described solutions, viability measurement based on 7-AAD staining and cytotoxic activity measurement were performed 1 and 3 hours after the thawing, and viability of the GAIA-102 cells was also analyzed after the reaction for the cytotoxic activity measurement.

### Results:

It was found that the activity could be maintained with (3) the solution consisting of SOLMALT and MEYLON at a ratio of 20:1, which was prepared on the basis of SOLMALT with pH adjustment with MEYLON. The activity-maintaining ability observed for the group (8) was comparable to that observed with (1) Plasma-Lyte A, and it was found that the addition of Imunace does not contribute to at least the activity for K562. (6) Klinisalz having a low pH, and (7) the solution containing Klinisalz/MEYLON, of which pH was adjusted, but which has a high osmolarity, could not maintain the activity (Fig. 7-1). High correlation between decrease in viability and the activity after replacement with RPMI was confirmed with good reproducibility (Fig. 7-2).

### 2-8) Narrowing down of factors affecting activity 3 hours after thawing 1

The GAIA-102 cells frozen for 48 hours or longer were thawed on a water bath at 37°C and then allowed to stand at room temperature up to 3 hours. Cytotoxicity activity was measured 3 hours after the thawing. After the thawing, the cells were diluted 41-fold with the solutions shown in the following table. There were prepared solutions of which pH was adjusted to 7.6 to 8.0 with MEYLON, and which was prepared to have a K⁺ concentration of 5 mEq/L, (1) a solution containing Albumin/K.C.L./physiological saline/MEYLON, and (2) a solution containing Albumin/K.C.L./physiological saline/MEYLON, further, for groups of a solution of which Cl⁻ concentration was further adjusted to 80 mMq/L, (3) a solution containing Albumin/K.C.L./physiological saline/Amizet/water/MEYLON, and (4) a solution containing K.C.L./physiological saline/Amizet/water/MEYLON, as well as (6) Pasma-Lyte A as a positive control. The pH values of the prepared solutions were also measured.

**[Table 7]**

| | 1 | 2 | 3 | 4 | 6 |
|---|---|---|---|---|---|
| | Albumin K.C.L. | Albumin K.C.L. Physiological saline MEYLON | Albumin K.C.L. Physiological saline Amizet Water MEYLON | K.C.L. Physiological saline Amizet Water MEYLON | Plasma-Lyte A |
| | MEYLON | | | | |
| Albumin | 500 mL | 250 mL | 23.4 mL | - | - |
| K.C.L. | 1.25 mL | 1.25 mL | 1.25 mL | 1.25 mL | - |
| Physiological saline | - | 250 mL | 234 mL | 244 mL | - |
| Amizet | - | - | 80 mL | 80 mL | - |
| Water | - | - | 162.6 mL | 176 mL | - |
| MEYLON | 37.6 mL | 25 mL | 16.7 mL | 16.7 mL | - |
| Plasma-Lyte A | - | - | - | - | 500 mL |
| pH | 7.7 | 7.7 | 7.7 | 7.8 | 7.2 |
| Osmolarity | 404.7 mOsm | 371.4 mOsm | 348.4 mOsm | 348.4 mOsm | |

### Results:

The solutions of (2) and (4) showed good activity-maintaining ability, and the solution of (3) showed markedly good results (Fig. 8).

### 2-9) Narrowing down of factors affecting activity 3 hours after thawing 2

The GAIA-102 cells frozen for 48 hours or longer were thawed on a water bath at 37°C and then allowed to stand at room temperature up to 4 hours. Cytotoxicity activity was measured 3 hours after the thawing, and viability measurement based on 7-AAD staining was performed 4 hours after the thawing. After the thawing, the cells were diluted 41-fold with the solutions shown in the following table. There were prepared (1) a solution containing Albumin (Albumin 25% I.V.-BENESIS, Japan Blood Products Organization)/K.C.L./physiological saline/Amizet/water/MEYLON, (2) a solution containing K.C.L./physiological saline/Amizet/water/MEYLON, (3) a solution containing Albumin/K.C.L./physiological saline/Amizet/water/MEYLON, and (5) Pasma-Lyte A as a positive control, all of which were prepared so as to have an osmolarity of 300 mOsm. The pH values of the prepared solutions were also measured.

**Table 8]**

| | 1 | 2 | 3 | 5 |
|---|---|---|---|---|
| | Albumin K.C.L. Physiological saline Amizet Water MEYLON | K.C.L. Physiological saline Amizet Water MEYLON | Albumin K.C.L. Physiological saline Amizet Water MEYLON | Plasma-Lyte A |
| Albumin | 29.66 mL | - | 220 mL | - |
| K.C.L. | 1.25 mL | 1.25 mL | 1.25 mL | - |
| Physiological saline | 296.60 mL | 308.44 mL | 220 mL | - |
| Amizet | 40.98 mL | 54.87 mL | 3.82 mL | - |
| Water | 128.76 mL | 132.71 mL | 52.18 mL | - |
| MEYLON | 4 mL | 2.74 mL | 4 mL | - |
| Plasma-Lyte A | - | - | - | 500 mL |
| PH | 7.3 | 7.3 | 7.3 | 7.2 |
| Osmolarity | 300 mOsm | 300 mOsm | 300 mOsm | 300 mOsm |

### Results:

The solution of (2) showed a good activity-maintaining ability, and the solutions of (1) and (3) showed markedly good results.

### 2-10) Statistical analysis

The data of the experiments conducted to date by the inventors of the present invention were compiled and statistically analyzed by using JMP (registered trademark) Pro 15 (SAS Institute Inc.). The results of the statistical analysis suggested that Cl⁻ concentration and pH significantly affect maintenance of the activity, and that there may be a threshold value for K⁺ (Fig. 10).

### 2-11) Use of another conditioned solution suitable for intravenous administration 1

The thawed GAIA-102 cells were diluted 41-fold with Plasma-Lyte A and the conditioned solution mentioned in the following table, and left to stand for 3 hours at room temperature, and cytotoxic activity against K562 and viability based on 7-AAD staining were measured.

The conditioned solution outperformed Plasma-Lyte A (Fig. 11).

**[Table 9]**

| |
|---|
| - Conditioned solution 200 mL |
| pH=7.2 |
| Albumin (Kenketsu Albumin) 25%: 11.112 mL |
| Physiological saline: 111.12 mL |
| Amizet: 19.08 mL |
| Water: 56.68 mL |
| MEYLON: 1.6 mL |
| K.C.L.: 0.4 mL |
| Osmolarity: 288 mOsm (calculated) |
| K⁺: 4 mEq/L |
| Na⁺: >99 mEq/L |
| Cl⁻: 93 mEq/L |
| pH: 7.3 |

### 2-12) Use of another conditioned solution suitable for intravenous administration 2

The thawed GAIA-102 cells were diluted 45-fold with Plasma-Lyte A, and the conditioned solution mentioned in the following table, which supposes addition of albumin upon use, and left to stand for 3 hours at room temperature, and then cytotoxic activity against K562 and viability based on 7-AAD staining were measured. The conditioned solution outperformed Plasma-Lyte A (Fig. 12).

**[Table 10]**

| |
|---|
| - Conditioned solution 33 mL |
| pH=7.3 |
| Albumin (Kenketsu Albumin) 25%: 3.00 mL |
| Physiological saline: 17.65 mL |
| Amizet: 3.03 mL |
| Water: 9.00 mL |
| MEYLON: 0.25 mL |
| K.C.L.: 0.06 mL |

### 3) Confirmation of effect of glucose

Frozen GAIA-102 cells were thawed on a water bath at 37°C for 2 minutes and 40 seconds, and then diluted 10-fold with each of the solutions for dilution.

**[Table 11]**

| Solutions for dilution |
|---|
| • Plasma-Lyte A (PL-A) |
| • PL-A + Glucose (1 g/L) |
| • PL-A + Glucose (2 g/L) |
| • PL-A + Glucose (4 g/L) |
| • PL-A + Glucose (8 g/L) |
| • PL-A + Glucose (16 g/L) |
| • PL-A + Glucose (26 g/L) |
| • PL-A + Glucose (38 g/L) |
| • PL-A + Glucose (50 g/L) |

After standing at room temperature for 3 hours, cytotoxic activity against K562 was evaluated. According to the method described in the section "E) Method for measuring cytotoxic activity against tumor cells", cytotoxic activity was measured with mixing the GAIA-102 cells (E) and K562 cells (T) at an ET ratio of 0.5:1, 1: 1, or 2:1. From the cytotoxic activity ratios calculated for ET ratios at 4 points (including 0), the calculated value for E:T=1:1 was obtained by nonlinear regression analysis using JMP (registered trademark) Pro statistical analysis software.

### Results:

High cytotoxic activity was maintained with the solutions for dilution having a glucose concentration not higher than 26 g/L, especially not higher than 16 g/L (Fig. 13).

## Claims

1. A solution for suspending cells for administration to humans, which satisfies the following criteria:
(1) containing potassium ions,
(2) having a pH of 6.4 or higher,
(3) not containing chloride ions at a concentration of 135 mEq/L or higher,
(4) not containing calcium ions at a concentration of 0.423 mM or higher, and
(5) having an osmolarity of 200 to 396 mOsm.

2. The solution according to claim 1, which is for diluting a frozen product containing cells for administration to humans or a thawed product thereof.

3. The solution according to claim 1, which
contains potassium ions at a concentration of 4.00 mEq/L or higher,
does not contain calcium ions, and
has a pH of 7.0 to 8.3.

4. A pharmaceutical composition containing a population of cells for administration to humans suspended in the solution according to any one of claims 1 to 3, wherein the cells for administration to humans are highly active NK cells.

5. The pharmaceutical composition according to claim 4, wherein the population of highly active NK cells has undergone a freezing step.

6. The pharmaceutical composition according to claim 5, wherein the population of highly active NK cells before freezing was collected by using a medium containing albumin, transferrin, insulin, and IL-2.

7. A method for providing a pharmaceutical composition for infusion containing cells for administration to humans, the method comprising:
(1) washing the cells with a medium for animal cell culture;
(2) optionally washing and suspending the cells in a cryopreservation solution;
(3) freezing and preserving (stocking) the suspended cells in the cryopreservation solution;
(4) thawing the cryopreserved cells; and
(5) suspending the thawed cells in the solution according to any one of claims 1 to 3 to prepare a pharmaceutical composition for infusion.
